# EUROPEAN PATENT APPLICATION

(11) **EP 4 814 015 A1**
(43) Date of publication of application: **30.09.2026**
(21) Application number: 24894396.1
(22) Date of filing: 24.10.2024
(51) Int. Cl.: C07K 16/28, A61P 37/08, A61P 29/00, A61P 19/10, A61P 33/06, G01N 33/50, G01N 33/68, A61K 39/00

(54) **CD300C, WHICH IS RECEPTOR OF DIMERIC TCTP, AND USE THEREOF**

(30) Priority: 20.11.2023 KR 20230161589
(71) Applicant: Ewha University-Industry Collaboration Foundation, Seoul 03760 (KR)
(72) Inventor: LEE, Kyung Lim, Seoul 03760 (KR); JANG, Eun-Hwa, Seoul 04104 (KR); BAE, Hae-Duck, Seoul 03760 (KR)
(74) Representative: Fuchs Patentanwälte Partnerschaft mbB
(86) International application number: PCT/KR2024/016336
(87) International publication number: WO 2025/110502

(57) **Abstract**

The present disclosure identifies for the first time that the receptor of dimeric TCTP is CD300c, and relates to an anti-CD300c antibody, a pharmaceutical use thereof, and a drug screening method and a disease diagnosis method using CD300c. In the present disclosure, it was confirmed that an extracellular domain of CD300c interacts with dimeric TCTP. Accordingly, CD300c can be effectively used as a target for the development of preventive or therapeutic agents for allergic diseases, inflammatory diseases, or malaria. The anti-CD300c antibody of the present disclosure can be effectively used for preventing or treating allergic diseases, inflammatory diseases, or malaria, or can be effectively used for drug screening or diagnosis related to allergic diseases, inflammatory diseases, or malaria.

## Description

### [Technical Field]

The present disclosure provides the first identification of CD300c as a receptor for dimeric translationally controlled tumor protein (TCTP), and relates to an anti-CD300c antibody, a pharmaceutical use thereof, and a drug screening method and a disease diagnosis method using CD300c.

### [Background Art]

Translationally controlled tumor protein (TCTP) is known as an IgE-dependent histamine-releasing factor that causes histamine to be released from basophils when specific IgE is present, but it was subsequently observed that TCTP regulates the secretion of histamine, IL-4, and IL-13 in inflammatory cells regardless of the presence of FcεR, an IgE receptor. In other words, while the possibility has been suggested that TCTP acts by binding to specific cell membrane receptors other than IgE, such receptors have not yet been identified (Blood 2000 Sep 15;96(6):2191-8).

The present inventors have confirmed through prior research that extracellularly secreted TCTP forms dimers, which activate bronchial epithelial cells and other inflammatory cells to secrete various cytokines and induce inflammatory responses. As a result, dimeric TCTP was proven for the first time to be an allergy-inducing substance (KR Patent No. 10-0780255; US Patent No. 7,772,368; JP Patent No. 4564926; EP Patent No. 1683866), and various studies have been conducted targeting dimeric TCTP for the treatment of related allergic and inflammatory diseases. As structural sites of dimeric TCTP, the inventors identified a flexible loop domain (KR Patent No. 10-1804291) or a helix 2 domain (KR Patent No. 10-1843051) and a C-terminus (KR Patent No. 10-1804285) that binds to the receptor of dimeric TCTP, and identified peptides, natural products, and monoclonal antibodies that bind to dimeric TCTP and have anti-allergic efficacy (KR Patent No. 10-1830838, KR Patent No. 10-1875317, and Korean Patent Application No. 10-2023-0087414).

Therefore, the present inventors identified CD300c, a receptor for dimeric TCTP associated with various diseases, and confirmed that inhibition of CD300c is capable of inhibiting the cytokine-like activity of dimeric TCTP. Therefore, the present inventors demonstrated that inhibiting CD300c provides preventative or therapeutic effects for allergic diseases, inflammatory diseases, and malaria, thereby completing the present disclosure.

### [Disclosure]

### [Technical Problem]

An object of the present disclosure is to provide an anti-CD300c antibody or an antigen-binding fragment thereof characterized by specifically binding to the extracellular domain of CD300c and inhibiting the binding of translationally controlled tumor protein (TCTP) to CD300c.

Another object of the present disclosure is to provide a use of the CD300c inhibitor for the prevention or treatment of allergic diseases, inflammatory diseases, or malaria.

Still another object of the present disclosure is to provide a nucleic acid molecule encoding the antibody or an antigen-binding fragment thereof, an expression vector comprising the same, and a host cell into which the expression vector is introduced.

Still another object of the present disclosure is to provide a drug screening method, a disease diagnosis method, and a diagnostic composition, each using CD300c.

### [Technical Solution]

The present disclosure is explained in detail as follows. Meanwhile, each description and embodiment disclosed in the present disclosure may be applied to each other description and embodiment. In other words, all combinations of the various elements disclosed in the present disclosure fall within the scope of the present disclosure. Furthermore, the scope of the present disclosure should not be considered limited by the specific descriptions described below.

To achieve the above objects, the present disclosure provides a pharmaceutical use of a CD300c inhibitor for the prevention or treatment of allergic diseases, inflammatory diseases, or malaria.

According to an embodiment of the present disclosure, the present disclosure provides a pharmaceutical composition for the prevention or treatment of allergic diseases, inflammatory diseases, or malaria, comprising a CD300c inhibitor as an active ingredient.

In addition, according to an embodiment of the present disclosure, the present disclosure provides a use of a CD300c inhibitor for use in the manufacture of a drug for the treatment or prevention of allergic diseases, inflammatory diseases, or malaria.

Further, according to an embodiment of the present disclosure, the present disclosure provides a method for preventing or treating allergic diseases, inflammatory diseases, or malaria, comprising administering a therapeutically effective amount of a CD300c inhibitor to a subject in need thereof.

As used herein, the term "CD300 antigen-like family member C (CD300c)" refers to a cell membrane protein also named CMRF35-like molecule 6 (CLM-6), etc. The present inventors confirmed that CD300c is the receptor for translationally controlled tumor protein (TCTP) dimers that directly affect diseases such as allergic diseases, inflammatory diseases, or malaria, thereby proving that CD300c is effectively applicable as a therapeutic target for the diseases. TCTP is a specific protein secreted outside the cell that is stored in small secretory endoplasmic reticulum and secreted via an atypical pathway, and this process is reportedly mediated by TSAP6, a p53-inducible membrane protein (Amzallag et al., J Biol Chem, 279, 46104-46112, 2004) and H,K-ATPase (Choi et al., PLoS One, 4(6), 5732-5739, 2009). It has been reported that these secreted TCTP dimers stimulate IgE-sensitized basophils to promote the release of histamine, interleukin-4, and interleukin-13, thereby causing late-phase response allergic diseases such as allergic rhinitis, asthma, and atopic dermatitis (MacDonald et al., Science, 269, 688-690, 1995).

In the present disclosure, it is confirmed through specific embodiments that CD300c may activate the NF-κB signaling pathway and secrete cytokines such as IL-6 and IL-8 through binding with TCTP dimers. Furthermore, it is confirmed that the signaling pathway and cytokine secretion are inhibited by an antibody that specifically binds to the extracellular domain of CD300c or by inhibiting the expression of CD300c via siRNA. Moreover, in an asthma mouse model, the inhibitory effect on airway inflammatory responses is confirmed by administering an antibody that specifically binds to the extracellular domain of CD300c, thereby reducing immune cell infiltration, mucus secretion, and cytokine secretion. Therefore, CD300c has sufficient potential to become a novel therapeutic target for various diseases previously known to be associated with TCTP.

As used herein, the term "CD300c inhibitor" is used to collectively refer to all agents that reduce the expression or activity of CD300c, and specifically, may include all agents that reduce the expression level or activity of CD300c by reducing its expression at the transcriptional level or interfering with its activity, such as by acting directly on CD300c or indirectly on its ligand. However, the CD300c inhibitor in the present disclosure does not indicate antibodies against TCTP.

The CD300c inhibitor may be used without limitation in its form, such as a compound, natural product, protein, nucleic acid, peptide, virus, or a vector containing the nucleic acid, which is able to target CD300c and inhibit the expression or activity of CD300c. The CD300c inhibitor may be, without limitation, an oligonucleotide that inhibits the expression of CD300c, or an antibody or its antigen-binding fragment that inhibits the activity of the CD300c protein. More specifically, the oligonucleotide that inhibits the expression of the CD300c may be an antisense oligonucleotide, shRNA, or siRNA, which is specific to CD300c mRNA, but is not limited thereto.

As used herein, the term "antisense oligonucleotide" refers to a DNA, RNA, or derivative thereof containing a nucleic acid sequence complementary to a specific mRNA sequence, which acts to inhibit the translation of the mRNA into a protein by binding to the complementary sequence within the mRNA. The antisense oligonucleotide sequence refers to a DNA or RNA sequence that is complementary to the CD300c mRNA and capable of binding to the mRNA. This may inhibit essential activities for the translation, translocation into the cytoplasm, maturation, or any other overall biological function of the CD300c mRNA. The antisense oligonucleotide may have a length of 6 to 100 bases, preferably 8 to 60 bases, and more preferably 10 to 40 bases. The above antisense oligonucleotide may be synthesized in vitro by conventional methods and administered into the body, or the antisense oligonucleotide may be synthesized in vivo. An example of synthesizing antisense oligonucleotides in vitro is to utilize RNA polymerase I. An example of synthesizing antisense RNA in vivo is to transcribe the antisense RNA using a vector in which the origin of the multiple cloning site (MCS) is oriented in the opposite direction. It is preferable that the antisense RNA be prevented from being translated into a peptide sequence by having a translation stop codon present in its sequence. The design of the antisense oligonucleotides to be usable in the present disclosure may be readily produced according to methods known in the art by referring to the nucleotide sequence of CD300c.

As used herein, the terms "siRNA" and "shRNA" refer to nucleic acid molecules capable of mediating RNA interference or gene silencing, which are used as efficient gene knockdown methods or gene therapy methods since these terms may suppress the expression of target genes. The shRNA refers to a single-stranded oligonucleotide that forms a hairpin structure via the binding between complementary sequences. Within a living body, the shRNA is cleaved by a dicer to become siRNA, a double-stranded oligonucleotide, which includes small RNA fragments of 21 to 25 nucleotides, and may specifically bind to mRNA having a complementary sequence to suppress expression. Therefore, the choice between shRNA and siRNA may be determined by those skilled in the art, and both cases may expect similar expression reduction effects when targeting identical mRNA sequences. For the purposes of the present disclosure, CD300c may be inhibited by acting specifically on CD300c to cleave CD300c mRNA molecules and inducing RNA interference (RNAi). The siRNA may be synthesized chemically or enzymatically. A method of preparing siRNA is not particularly limited and may be any method known in the art. Examples thereof may include, but are not limited to, methods of directly synthesizing siRNA chemically, methods of synthesizing siRNA using in vitro transcription, methods of cleaving long double-stranded RNA synthesized by in vitro transcription using enzymes, methods of expression through intracellular delivery of shRNA expression plasmids or viral vectors, and methods of expression through intracellular delivery of PCR (polymerase chain reaction)-induced siRNA expression cassettes.

In addition, the antibody or its antigen-binding fragment that inhibits the activity of the CD300c protein in the present disclosure may be characterized by specifically binding to the extracellular domain of CD300c (e.g., amino acids 1 to 183 of SEQ ID NO: 3 or amino acids 22 to 188 of the amino acids of SEQ ID NO: 56) and inhibiting the binding of translationally controlled tumor protein (TCTP) to CD300c.

As used herein, the term "antibody" refers to a protein molecule that acts as a receptor for specifically recognizing an antigen, comprising an immunoglobulin molecule having immunological reactivity with a specific antigen, and may include, for example, monoclonal antibodies, polyclonal antibodies, full-length antibodies, and antibody fragments. In addition, the term may include bivalent or bispecific molecules (e.g., bispecific antibodies), diabodies, triabodies, or tetrabodies.

As used herein, the term "monoclonal antibody" refers to an antibody molecule of a single molecular composition obtained from a population of substantially homogeneous antibodies, and these monoclonal antibodies exhibit single-binding specificity and affinity for specific epitopes. As used herein, the term "full-length antibody" refers to a structure having two full-length light chains and two full-length heavy chains, where each light chain is connected to a heavy chain by a disulfide bond. The heavy chain constant region has gamma (γ), mu (µ), alpha (α), delta (δ), and epsilon (ε) types and has gamma 1 (γ1), gamma 2 (γ2), gamma 3 (γ3), gamma 4 (γ4), alpha 1 (α1), and alpha 2 (α2) subclasses. The light chain constant region has kappa (κ) and lambda (λ) types. IgG has subtypes including IgG1, IgG2, IgG3, and IgG4.

As used herein, the terms "fragment," "antibody fragment," and "antigen-binding fragment" are used interchangeably to refer to any fragment of the antibody of the present disclosure that retains the antigen-binding function of the antibody. Exemplary antigen-binding fragments include, but are not limited to, Fab, Fab', F(ab')₂, and Fv.

The Fab has a structure having a variable region of the light chain and heavy chain, a constant region of the light chain, and a first constant region (CH1 domain) of the heavy chain, and has one antigen-binding site. An antigen-binding fragment or antibody fragment of an antibody molecule refers to a fragment that retains antigen-binding function. Fab' differs from Fab in that it includes a hinge region containing one or more cysteine residues at the C-terminus of the CH1 domain of the heavy chain. An F(ab')₂ antibody is formed when the cysteine residues in the hinge regions of Fab' fragments form disulfide bonds. Fv is the smallest antibody fragment, consisting only of the heavy chain variable region and the light chain variable region. Recombinant techniques for producing Fv fragments are disclosed in PCT International Patent Publications WO 88/10649, WO 88/106630, WO 88/07085, WO 88/07086, and WO 88/09344. A two-chain Fv includes a heavy variable region and a light variable region connected by non-covalent bonds, while a single-chain Fv generally includes the heavy chain and light chain variable regions covalently linked via a peptide linker or directly joined at the C-terminus to form a dimer-like structure similar to the two-chain Fv. Although not limited to thereto, these antibody fragments may be obtained using proteolytic enzymes (e.g., Fab fragments may be obtained by restrictive cleavage of a whole antibody with papain, and F(ab')₂ fragments may be obtained by cleavage with pepsin) or may be produced through recombinant DNA technology.

As used herein, the allergic disease may be asthma, bronchitis, chronic obstructive pulmonary disease, bronchiectasis, rhinitis, atopic dermatitis, urticaria, hay fever, conjunctivitis, or anaphylaxis, but is not limited thereto.

As used herein, the inflammatory disease may be Rheumatoid arthritis, bronchitis, pneumonia, arthritis, nephritis, psoriasis, dermatitis, Crohn's disease, enteritis, gingivitis, arteriosclerosis, coronary arteritis, hepatitis, Behcet's disease, bladder cancer, prostatitis, pyelonephritis, glomerulonephritis, osteomyelitis, thyroiditis, uveitis, peritonitis, meningitis, or pulmonary fibrosis, but is not limited thereto.

IL-8, which is increased by TCTP, is known to be involved in various allergic diseases such as asthma or bronchitis (Chanez et al., Int Arch Allergy Immunol, 111, 83-88, 1996), chronic obstructive pulmonary disease (Nocker et al., Int Arch Allergy Immunol, 109, 183-191, 1996), bronchiectasis (Simpson et al., Thorax, 62, 211-218, 2007), rhinitis (Benson et al., Pediatr Allergy Immunol, 10, 178-185, 1999; Kuna et al., J Allergy Clin Immunol, 97, 104-112, 1996), atopic dermatitis (Kimata & Lindley, Arch Dis Child 70, 119-122, 1994), urticaria (Choi et al., J Clin Immunol, 28, 244-249, 2008), hay fever (Ciprandi et al., Otolaryngol Head Neck Surg, 133, 429-435, 2005), conjunctivitis (Miyoshi et al., Cornea, 20, 743-747, 2001), anaphylaxis (Cho et al., Front Pharmacol., 12, 764321-764329, 2021), etc.

In addition, IL-8 is known to be involved in various inflammatory diseases such as chronic inflammatory bronchial diseases such as chronic bronchitis (Richman-Eisenstat et al., Am J Physiol, 264, L413-418, 1993), inflammatory lung diseases such as pneumonia (Erger and Casale, Eur Respir J, 11, 299-305, 1998; Pease & Sabroe, Am J Respir Med, 1, 19-25, 2002), arthritis or nephritis (Harada et al., J Leukoc Biol, 56, 559-564, 1994), psoriasis (Schulz et al., J Immunol, 151, 4399-4406, 1993; Bruch-Gerharz et al., J Exp Med, 184, 2007-2012, 1996), dermatitis (Sticherling et al., Arch Dermatol Res, 284, 82-85, 1992), Crohn's disease (Izutani et al., Inflamm Bowel Dis, 1, 37-47, 1995), inflammatory bowel disease (Mitsuyama et al., Clin Exp Immunol, 96, 432-436, 1994), gingivitis (Haake & Huang, Clinical Periodontology, 9th Edition. Philadelphia: W.B.Saunders Co. 2002. page 162), cardiovascular diseases such as arteriosclerosis and coronary artery disease (Apostolakis et al., Cardiovasc Res, 84, 353-360, 2009; Boekholdt et al., Arterioscler Thromb Vasc Biol, 24, 1503-1508, 2004), chronic liver disease (Zimmermann et al., PLoS ONE, 6, e21381, 2011), Behcet's disease (Behcet's disease, Katsantonis et al., Dermatology, 201, 37-39, 2000), bladder cancer, prostatitis, pyelonephritis, or osteomyelitis (Shahzad et al., Int arch med, 3, 11, 2010), thyroid disease (Kobawala et al., J Thyroid Res, 8, 270149, 2011), uveitis (uveitis, Klok et al., Br J Ophthalmol, 82, 871-874, 1998), glomerulonephritis, peritonitis, meningitis, and pulmonary fibrosis (Harada et al., Mol Med Today, 2, 482-489, 1996). Therefore, the inhibition of IL-8 has been suggested as a therapeutic strategy for inflammatory diseases such as lung disease, rheumatoid arthritis, inflammatory bowel disease, and chronic inflammatory skin diseases such as psoriasis and palmoplantar pustulosis, as well as ocular inflammation (Mukaida, Am J Physiol Lung Cell Mol Physiol, 284, L566-L577, 2003; Skov et al., J Immunol, 181, 669-679, 2008; Harada et al., J Leukoc Biol, 56, 559-564, 1994). Therapies that utilize IL-8 blocking antibodies or inhibit genes encoding IL-8 receptors have demonstrated antiinflammatory effects (Harada et al., Mol Med Today, 2, 482-489, 1996), and for example, the administration of antibodies against IL-8 resulted in a reduction of inflammation in patients with chronic inflammatory skin diseases (Skov et al., J Immunol, 181, 669-679, 2008). GM-CSF, whose secretion is increased by TCTP, is also associated with various inflammatory diseases (Hamilton, Trends Immunol, 23, 403-408, 2002), and is therefore suggested as a target for inflammatory diseases such as rheumatoid arthritis (Cornish et al., Nat Rev Rheumatol, 5, 554-559, 2009).

In addition, it was revealed in 1998 that the antimalarial drug artemisinin acts by binding to TCTP, a malaria protein (Bhisutthibhan et al., J Biol Chem, 273, 16192-16198, 1998). IL-8 is also secreted in malaria patients (Friedland et al., Trans R Soc Trop Med Hyg, 87, 54-55, 1993), and it has been reported that malaria HRF promotes IL-8 secretion (MacDonald et al., Proc Natl Acad Sci U S A, 98, 10829-32, 2001).

The results of these prior studies demonstrate that various allergic diseases, inflammatory diseases, and malaria are capable of being prevented and treated by targeting CD300c, the receptor for TCTP.

As used herein, the term "treatment" refers to clinical intervention to alter the natural processes of an individual or cell to be treated, which may be performed either during the progression of a clinical pathological condition or for its prevention. The intended therapeutic effects include preventing the onset or recurrence of the disease, alleviating symptoms, reducing all direct or indirect pathological consequences associated with the disease, preventing metastasis, reducing the rate of disease progression, alleviating or temporarily relieving the disease state, inducing remission, or improving the prognosis. Preferably, the present disclosure includes all actions that improve the course of the disease by administering a composition containing a substance that inhibits CD300c. Furthermore, "prevention" refers to all actions that suppress or delay the onset of the disease by administering a composition containing a substance that inhibits CD300c according to the present disclosure.

The pharmaceutical composition of the present disclosure may further comprise a suitable carrier, excipient, or diluent commonly used in the preparation of pharmaceutical compositions. A composition comprising a pharmaceutically acceptable carrier may be in various dosage forms for oral or parenteral administration. For formulation, preparations may be made using commonly used diluents or excipients such as commonly used fillers, expanders, binders, wetting agents, disintegrants, and surfactants. Solid dosage forms for oral administration may include tablets, powders, granules, capsules, etc., and these solid dosage forms may be prepared by mixing at least one excipient, for example, starch, calcium carbonate, sucrose or lactose, gelatin, etc., with one or more compounds. In addition, lubricants such as magnesium stearate and talc may be used in addition to simple excipients. Liquid preparations for oral administration may include suspensions, oral liquids, emulsions, and syrups. In addition to commonly used simple diluents such as water and liquid paraffin, various excipients, such as wetting agents, sweeteners, flavoring agents, and preservatives, may also be included. Preparations for parenteral administration may include sterile aqueous solutions, non-aqueous solvents, suspensions, emulsions, lyophilized preparations, and suppositories. As non-aqueous solvents and suspension solvents, propylene glycol, polyethylene glycol, vegetable oils such as olive oil, and injectable esters such as ethyl oleate may be used. As bases for suppositories, Witepsol, Macrogol, Tween 61, cacao butter, laurin fat, glycerogelatin, etc., may be used.

In addition, the pharmaceutical composition of the present disclosure may have, but is not limited to, any one formulation selected from the group consisting of tablets, pills, powders, granules, capsules, suspensions, oral liquids, emulsions, syrups, sterile aqueous solutions, non-aqueous solvents, suspensions, emulsions, lyophilized preparations, and suppositories.

Another aspect of the present disclosure for achieving the above object is an anti-CD300c antibody or an antigen-binding fragment thereof that specifically binds to the extracellular domain of CD300c (amino acids 1 to 183 of SEQ ID NO: 3 or amino acids 22 to 188 of the amino acids of SEQ ID NO: 56) and inhibits the binding of translationally controlled tumor protein (TCTP) to CD300c.

According to an embodiment of the present disclosure,
(a) the anti-CD300c antibody or an antigen-binding fragment thereof may comprise: a light chain variable region comprising a light chain CDR1 set forth in SEQ ID NO: 9 or SEQ ID NO: 34, a light chain CDR2 set forth in SEQ ID NO: 10 or SEQ ID NO: 35, and a light chain CDR3 set forth in SEQ ID NO: 11 or SEQ ID NO: 36; and a heavy chain variable region comprising a heavy chain CDR1 set forth in SEQ ID NO: 12 or SEQ ID NO: 27, a heavy chain CDR2 set forth in SEQ ID NO: 13 or SEQ ID NO: 28, and a heavy chain CDR3 set forth in SEQ ID NO: 14, SEQ ID NO: 22, SEQ ID NO: 44, or SEQ ID NO: 51; or
(b) the anti-CD300c antibody or an antigen-binding fragment thereof may comprise at least one amino acid substitution in the sequences of (a), wherein: in the heavy chain CDR1 set forth in SEQ ID NO: 27, F at position 2 is substituted with Y, N at position 3 is substituted with T, R at position 5 is substituted with G, T, or S, N at position 6 is substituted with G or S, or A at position 8 is substituted with G; in the heavy chain CDR2 set forth in SEQ ID NO: 28, N at position 2 is substituted with S, G at position 3 is substituted with W or V, R at position 4 is substituted with N, Y, or S, G at position 5 is substituted with N, D at position 6 is substituted with G, T at position 7 is substituted with D, N, or S, or T at position 8 is substituted with I; or in the heavy chain CDR3 set forth in SEQ ID NO: 51, R at position 2 is substituted with S, G at position 3 is substituted with E, P at position 4 is substituted with A or D, Y at position 5 is substituted with S, Y at position 6 is substituted with SS, F at position 7 is substituted with Y, or D at position 8 is substituted with N; and wherein the heavy chain CDR1 comprises 0 to 5 substitutions, the heavy chain CDR2 comprises 0 to 6 substitutions, and the heavy chain CDR3 comprises 0 to 4 substitutions, provided that heavy chain CDR1, heavy chain CDR2, or heavy chain CDR3 comprises at least one substitution.

In the present disclosure, the term "heavy chain" may include both a full-length heavy chain and fragments thereof, comprising a variable region domain VH containing an amino acid sequence having a sufficient variable region sequence to confer specificity to an antigen and three constant region domains CH1, CH2, and CH3. Further, in the present disclosure, the term "light chain" may include both a full-length light chain and fragments thereof, comprising a variable region domain VL containing an amino acid sequence having a sufficient variable region sequence to confer specificity to an antigen and a constant region domain CL.

In the present disclosure, the antibody may include both mouse-derived antibodies and variants thereof, in which a portion of the amino acid sequence of the parent antibody is modified by substitution, addition, and/or deletion to improve affinity, immunogenicity, etc. The variants are not limited thereto, but may include, for example, chimeric antibodies, humanized antibodies, affinity-optimized antibodies, etc.

In the present disclosure, the above variants comprehensively refer to antibodies in which a portion of the amino acid sequence of the parent antibody CDR is mutated (substituted, added, or deleted) under conditions that include the same CDR as the parent antibody or target the same epitope. Such variants may be appropriately adjusted by those skilled in the art to improve the affinity and immunogenicity of the antibody within a range in which binding ability to the same epitope is maintained.

In other words, the antibody of the present disclosure or antigen-binding fragment thereof may include not only the sequence of the anti-CD300c antibody described herein, but also biological equivalents thereof, within a range capable of specifically recognizing CD300c. For example, additional modifications may be made to the antibody's amino acid sequence to further improve its binding affinity and other biological properties. Such modifications include the deletion, insertion, and/or substitution of amino acid sequence residues of the antibody. These amino acid variations are made based on the relative similarities of amino acid side chain substituents, such as hydrophobicity, hydrophilicity, charge, size, etc. Based on an analysis of the size, shape, and type of amino acid side chain substituents, arginine, lysine, and histidine are all positively charged residues; alanine, glycine, and serine are similar in size; and phenylalanine, tryptophan, and tyrosine share similar structural shapes. Therefore, based on these considerations, arginine, lysine, and histidine; alanine, glycine, and serine; and phenylalanine, tryptophan, and tyrosine may be considered biologically functional equivalents.

In the present disclosure, the term "chimeric antibody" refers to an antibody formed by recombining the variable region of a mouse antibody and the constant region of a human antibody, and exhibits significantly reduced immunogenicity compared to the mouse antibody.

As used herein, the term "humanized antibody" refers to an antibody in which the protein sequence of an antibody derived from a non-human species is modified to be similar to a variant of an antibody naturally produced in humans. For example, the humanized antibody may be produced by recombining a mouse-derived CDR with a human antibody-derived FR to produce a humanized variable region, which is then recombined with the constant region of a desired human antibody. However, since simple CDR grafting alone often leads to a decrease in the affinity of the humanized antibody, the affinity may be restored to the level of the original mouse antibody by substituting several critical FR amino acid residues, expected to influence the three-dimensional structure of the CDRs, with those from the mouse antibody.

In an embodiment of the present disclosure, the antibody may comprise, but is not limited to, a light chain variable region comprising a light chain CDR1 set forth in SEQ ID NO: 9 or SEQ ID NO: 34, a light chain CDR2 set forth in SEQ ID NO: 10 or SEQ ID NO: 35, and a light chain CDR3 set forth in SEQ ID NO: 11 or SEQ ID NO: 36; and a heavy chain variable region comprising a heavy chain CDR1 set forth in SEQ ID NO: 12, SEQ ID NO: 20, SEQ ID NO: 27, SEQ ID NO: 37 or SEQ ID NO: 49, a heavy chain CDR2 set forth in SEQ ID NO: 13, SEQ ID NO: 21, SEQ ID NO: 28, SEQ ID NO: 38 or SEQ ID NO: 50, and a heavy chain CDR3 set forth in SEQ ID NO: 14, SEQ ID NO: 22, SEQ ID NO: 29, SEQ ID NO: 39, SEQ ID NO: 44 or SEQ ID NO: 51.

In an embodiment of the present disclosure, the antibody may comprise, but is not limited to, a light chain variable region set forth in SEQ ID NO: 7, SEQ ID NO: 18, SEQ ID NO: 25, SEQ ID NO: 32, SEQ ID NO: 42, SEQ ID NO: 47 or SEQ ID NO: 54; and a heavy chain variable region set forth in SEQ ID NO: 8, SEQ ID NO: 19, SEQ ID NO: 26, SEQ ID NO: 33, SEQ ID NO: 43, SEQ ID NO: 48 or SEQ ID NO: 55.

In another embodiment of the present disclosure, the antibody may further comprise a linker set forth in SEQ ID NO: 15, and specifically, may consist of the amino acid sequence set forth in SEQ ID NO: 6, SEQ ID NO: 17, SEQ ID NO: 24, SEQ ID NO: 31, SEQ ID NO: 41, SEQ ID NO: 46, or SEQ ID NO: 53, but is not limited thereto.

According to an embodiment of the present disclosure,
the antibody or an antigen-binding fragment thereof may comprise a light chain variable region comprising a light chain CDR1 set forth in SEQ ID NO: 61,
a light chain CDR2 set forth in SEQ ID NO: 62, and
a light chain CDR3 set forth in SEQ ID NO: 63; and
a heavy chain variable region comprising a heavy chain CDR1 set forth in SEQ ID NO: 64,
a heavy chain CDR2 set forth in SEQ ID NO: 65, and
a heavy chain CDR3 set forth in SEQ ID NO: 66.

In an embodiment of the present disclosure, the antibody may comprise a light chain variable region set forth in SEQ ID NO: 59 and a heavy chain variable region set forth in SEQ ID NO: 60, but is not limited thereto.

In another embodiment of the present disclosure, the antibody may further comprise a linker set forth in SEQ ID NO: 15, and specifically, may consist of the amino acid sequence set forth in SEQ ID NO: 58, but is not limited thereto.

In another general aspect, the present disclosure provides a nucleic acid molecule encoding the antibody or an antigen-binding fragment thereof, an expression vector comprising the nucleic acid molecule, and a host cell into which the expression vector is introduced.

The above-described antibody and antigen-binding fragment thereof are as described above.

The term "nucleic acid molecule" as used herein comprehensively includes DNA and RNA molecules, and the nucleotide, which is the basic structural unit of the nucleic acid molecule, includes not only natural nucleotides but also analogues in which the sugar or base region is modified (Scheit, Nucleotide Analogs, John Wiley, New York(1980); Uhlman and Peyman, Chemical Reviews, (1990) 90:543-584). The sequence of a nucleic acid molecule encoding the heavy and light chain variable regions of the present disclosure may be modified, and the modification includes the addition, deletion, or non-conservative or conservative substitution of nucleotides.

The nucleic acid molecule of the present disclosure is interpreted to also include a nucleotide sequence that exhibits substantial identity with respect to the nucleotide sequence described above. As used herein, substantial identity means a nucleotide sequence that exhibits at least 80% homology, specifically at least 90% homology, and more specifically at least 95% homology when the nucleotide sequence of the present disclosure described above is aligned with any other sequence to the greatest extent possible and the aligned sequence is analyzed using an algorithm commonly used in the art.

As used herein, the term "vector" includes, but is not limited to, plasmid vectors; cosmid vectors; and viral vectors such as bacteriophage vectors, adenovirus vectors, retrovirus vectors, and adeno-associated virus vectors as means for expressing a target gene in a host cell, and specifically may be a plasmid vector, but is not limited thereto.

In the vector of the present disclosure, the nucleic acid molecule encoding the light chain variable region and the nucleic acid molecule encoding the heavy chain variable region may be operably linked to a promoter.

As used herein, the term "operably linked" means a functional linkage between a nucleic acid expression regulatory sequence (e.g., a promoter, a signal sequence, or an array of transcription factor binding sites) and another nucleic acid sequence, and thus the regulatory sequence controls the transcription and/or translation of the other nucleic acid sequence.

The recombinant vector system of the present disclosure may be constructed through various methods known in the art. For example, specific methods for this system are disclosed in Sambrook et al., Molecular Cloning, A Laboratory Manual, Cold Spring Harbor Laboratory Press (2001), which is incorporated herein by reference.

The vector of the present disclosure may typically be constructed as a vector for cloning or as a vector for expression. Further, the vector of the present disclosure may be constructed using a prokaryotic cell or a eukaryotic cell as a host.

For example, when the vector of the present disclosure is an expression vector and a prokaryotic cell serves as the host, the vector typically includes a strong promoter capable of initiating transcription (e.g., tac promoter, lac promoter, lacUV5 promoter, lpp promoter, pLλ promoter, pRλ promoter, rac5 promoter, amp promoter, recA promoter, SP6 promoter, trp promoter, and T7 promoter, etc.), a ribosome binding site for initiating translation, and a transcription/translation termination sequence. When E. coli (e.g., HB101, BL21, DH5α, etc.) is used as a host cell, the promoter and operator sites of the E. coli tryptophan biosynthetic pathway (Yanofsky, C., J. Bacteriol., (1984) 158:1018-1024), and the leftward promoter of phage λ (pLλ promoter, Herskowitz, I. and Hagen, D., Ann. Rev. Genet., (1980) 14:399-445) may be used as regulatory sites. When Bacillus is used as a host cell, the promoter of the toxin protein gene of *Bacillus thuringiensis* (Appl. Environ. Microbiol. (1998) 64:3932-3938; Mol. Gen. Genet. (1996) 250:734-741) or any promoter capable of being expressed in Bacillus may be used as a regulatory site.

Meanwhile, the recombinant vector of the present disclosure may be produced by manipulating plasmids (e.g., pCL, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19, etc.) commonly used in the art, phages (e.g., λgt4•λB, λ-Charon, λΔz1 and M13, etc.), or viruses (e.g., SV40, etc.). For example, the recombinant vector of the present disclosure may be produced by manipulating a pCL expression vector, specifically the pCLS05 expression vector (KR Patent No. 10-1420274), but is not limited thereto.

Meanwhile, when the vector of the present disclosure is an expression vector and a eukaryotic cell serves as the host, a promoter derived from the genome of a mammalian cell (e.g., metallothionein promoter, β-actin promoter, human hemoglobin promoter, and human muscle creatine promoter) or a promoter derived from a mammalian virus (e.g., adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, and Rous sarcoma virus (RSV) promoter) may be used, and the vector generally has a polyadenylation sequence as a transcription termination sequence. Specifically, the recombinant vector of the present disclosure includes a CMV promoter.

The recombinant vector of the present disclosure may be fused with other sequences to facilitate the purification of the antibody expressed therefrom. The fused sequences include, for example, glutathione S-transferase (Pharmacia, USA), maltose binding protein (NEB, USA), FLAG (IBI, USA), and 6x His (hexahistidine; Qiagen, USA). Furthermore, since the protein expressed by the vector of the present disclosure is an antibody, the expressed antibody may be easily purified through a protein A column, etc., without the need for additional sequences for purification.

Meanwhile, the recombinant vector of the present disclosure includes antibiotic resistance genes commonly used in the art as selection markers, and may include, for example, resistance genes to ampicillin, gentamicin, carbenicillin, chloramphenicol, streptomycin, kanamycin, geneticin, neomycin, and tetracycline.

The vector expressing the antibody of the present disclosure may be a vector system in which the light chain and heavy chain are simultaneously expressed in a single vector, or a system in which the light chain and heavy chain are expressed in separate vectors. In the latter case, the two vectors may be introduced into host cells, for example, through co-transformation or targeted transformation. Co-transformation is a method of simultaneously introducing the respective vector DNA encoding the light chain and the heavy chain into host cells, followed by selecting cells that express both chains. Meanwhile, targeted transformation is a method of selecting cells transformed with a vector containing the light chain (or heavy chain) and then transforming the selected cells again with a vector containing the heavy chain (or light chain) to finally select cells that express both the light chain and the heavy chain.

Any host cell known in the art capable of achieving stable and continuous cloning and expression of the vector of the present disclosure may be used, and may include, for example, but is not limited to, prokaryotic host cells such as strains of the genus Escherichia (e.g., *Escherichia coli*), the genus Bacillus (e.g., *Bacillus subtilis* and *Bacillus thuringiensis*), Streptomyces, Pseudomonas (e.g., *Pseudomonas putida*), Proteus mirabilis, or Staphylococcus (e.g., *Staphylococcus carnosus*).

Suitable eukaryotic host cells for the above vector may include fungi such as *Aspergillus species*, yeasts such as *Pichia pastoris*, *Saccharomyces cerevisiae*, *Schizosaccharomyces*, and *Neurospora crassa*, other lower eukaryotic cells, cells from higher eukaryotes such as insect-derived cells, and cells derived from plants or mammals.

Specifically, the host cell may be COS7 cells (monkey kidney cells), NSO cells, SP2/0, Chinese hamster ovary (CHO) cells, W138, baby hamster kidney (BHK) cells, MDCK, myeloma cell line, HuT 78 cells, or 293 cells. More specifically, the host cell may be a Chinese hamster ovary (CHO) cell, but is not limited thereto.

In the present disclosure, "transformation" and/or "transfection" into a host cell include any method of introducing nucleic acid into an organism, cell, tissue, or organ, and may be performed by selecting a standard technique suitable for the host cell as known in the art. The methods include, but are not limited to, electroporation, protoplasmic fusion, calcium phosphate (CaPO₄) precipitation, calcium chloride (CaCl₂) precipitation, stirring using silicon carbide fibers, Agrobacterium-mediated transformation, PEG, dextran sulfate, lipofectamine, and drying/inhibition-mediated transformation methods.

In another general aspect, there is provided a drug screening method for the prevention or treatment of allergic diseases, inflammatory diseases, or malaria, comprising: (a) treating isolated cells expressing CD300c with dimeric translationally controlled tumor protein (dTCTP) and a candidate substance; (b) measuring the level of NF-κB signaling pathway activity or IL-8 secretion in the isolated cells treated with the candidate substance; and (c) screening the candidate substance that reduces the level of NF-κB signaling pathway activity or IL-8 secretion by comparing the level of NF-κB signaling pathway activity or IL-8 secretion measured in step (b) above with the level in isolated cells not treated with the candidate substance. Here, the expression of CD300c includes both naturally expressed CD300c and artificially induced CD300c.

Specifically, the level of activity of the NF-κB signaling pathway or IL-8 secretion in cells may be measured in the absence of a candidate substance capable of preventing or treating allergic diseases, inflammatory diseases, or malaria, and the level of activity of the signaling pathway or IL-8 secretion in the presence of the candidate substance may also be measured and compared. Then, the substance that reduces the level of activity of the signaling pathway or IL-8 secretion in the presence of the candidate substance compared to the level in the absence of the candidate substance may be predicted as an agent for the prevention or treatment of allergic diseases, inflammatory diseases, or malaria.

The candidate substance may be a known substance or a novel substance, and may be screened on a large scale, such as through plant extracts or a chemical library.

In still another general aspect, there is provided a method for providing information for the diagnosis of allergic diseases, inflammatory diseases, or malaria, comprising: (a) measuring the expression or secretion level of CD300c from an isolated biological sample; and (b) comparing the measured expression or secretion level with the CD300c expression or secretion level of a normal control sample.

In still another general aspect, there is provided a composition for the diagnosis of allergic diseases, inflammatory diseases, or malaria, comprising an agent for measuring the expression or secretion level of CD300c.

As confirmed through specific embodiments of the present disclosure, treatment with dimeric TCTP increases the expression of CD300c, and conversely, inhibition of the expression or activity of CD300c reduces the activity of signaling pathways and cytokine secretion induced by dimeric TCTP, thereby enabling the diagnosis of TCTP-associated diseases, namely allergic diseases, inflammatory diseases, or malaria, by confirming the level of expression or secretion of CD300c from isolated biological samples.

As used herein, the term "diagnosis" refers to identifying the presence or determining the characteristics of an allergic disease, an inflammatory disease, or malaria by measuring the presence or absence of the CD300c of the present disclosure in a biological sample or tissue sample. Further, "marker or diagnosis marker" refers to a substance capable of diagnosing allergic diseases, inflammatory diseases, or malaria by distinguishing subjects having the diseases from normal cells or healthy subjects, and includes organic biomolecules such as polypeptides, proteins or nucleic acids (e.g., mRNA, etc.), lipids, glycolipids, glycoproteins, or sugars (monosaccharides, disaccharides, oligosaccharides, etc.) that show an increase or decrease in cells or subjects with an allergic disease, an inflammatory disease, or malaria compared to normal cells. For the purposes of the present disclosure, the diagnostic marker for allergic disease, inflammatory disease, or malaria of the present disclosure is CD300c, which exhibits specifically high levels of expression in cells affected by allergic diseases, inflammatory diseases, or malaria compared to normal cells or cells of tissue.

In the present disclosure, measuring the CD300c expression level may involve measuring the mRNA expression level of CD300c or measuring the CD300c protein expression level.

The term "measuring mRNA expression level" refers to a process of confirming the presence and degree of expression of mRNA of an allergic disease, inflammatory disease, or malaria marker gene in a biological sample to diagnose allergic diseases, inflammatory diseases, or malaria, which may be determined by measuring the amount of mRNA. Analysis methods for measuring the mRNA expression level include, but are not limited to, RT-PCR, competitive RT-PCR, real-time RT-PCR, RNase protection assay (RPA), Northern blotting, and DNA chips.

The term "measuring protein expression level" refers to a process of confirming the presence and degree of expression of proteins expressed in allergy disease, inflammatory disease, or malaria marker genes in biological samples to diagnose allergic diseases, inflammatory diseases, or malaria, which may be determined by confirming the amount of protein using an antibody that specifically binds to the protein of the gene. Analytical methods for measuring the protein expression level include, but are not limited to, Western blot, enzyme linked immunosorbent assay (ELISA), radioimmunoassay (RIA), radioimmunodiffusion, Ouchterlony immunodiffusion, rocket immunoelectrophoresis, tissue immunostaining, immunoprecipitation assay, complement fixation assay, FACS, and protein chips.

For example, the agent for measuring the mRNA level may be a primer pair, a probe, or an antisense nucleotide for the mRNA of CD300c of the present disclosure, and a person skilled in the art may easily design the primer, probe, or antisense nucleotide sequences based on the polynucleotide sequence of CD300c of the present disclosure. As another example, the agent for measuring the protein level may be an antibody.

Embodiments of the present disclosure may be modified in various different forms, and the scope of the present disclosure is not limited to the embodiments described below. Furthermore, these embodiments are provided to fully convey the spirit of the invention to a person having ordinary skill in the art. Furthermore, throughout this specification, the term "comprising" implies the inclusion of stated components without the exclusion of any other elements unless specifically stated otherwise.

### [Advantageous Effects]

In the present disclosure, it was confirmed that the extracellular domain of CD300c interacts with dimeric TCTP, and accordingly, CD300c can be usefully utilized in the development of preventive or therapeutic agents for allergic diseases, inflammatory diseases, or malaria. Further, the anti-CD300c antibody of the present disclosure can be effectively used for preventing or treating allergic diseases, inflammatory diseases, or malaria, or can be effectively used for drug screening or diagnosis related to allergic diseases, inflammatory diseases, or malaria.

### [Description of Drawings]

FIG. 1 shows the results of Western blot analysis of the interaction between the CD300c extracellular domain or control protein and dimeric or monomeric TCTP via pull-down analysis.
FIG. 2 is a graph showing IL-8 secretion ability in bronchial epithelial cells treated after preincubation of dimeric TCTP and various concentrations of the CD300c extracellular domain.
FIG. 3 shows the results of Western blot analysis of changes in CD300c expression over time after treating bronchial epithelial cells with dimeric TCTP.
FIG. 4a shows the Western blot results of the knockdown efficiency of CD300c in bronchial epithelial cells.
FIG. 4b is a graph showing comparison of cytokine secretion capacity by dimeric TCTP treatment following the knockdown of CD300c in bronchial epithelial cells.
FIG. 5 shows the results of measuring NF-κB activity induced by dimeric TCTP treatment following CD300c overexpression in a cell line expressing an NF-κB activity-dependent reporter gene.
FIG. 6a is a graph showing the primary screening results confirming the inhibitory effect of the anti-CD300c scFv-Fc antibody against dimeric TCTP in bronchial epithelial cells.
FIG. 6b is a graph showing comparison of the binding strengths of four types of anti-CD300c scFv-Fc antibodies to the CD300c protein, as determined by ELISA.
FIG. 7a is a graph showing the secondary screening results confirming the inhibitory effect of the anti-CD300c scFv-Fc antibody against dimeric TCTP in bronchial epithelial cells.
FIG. 7b is a graph showing comparison of the binding strengths of three types of anti-CD300c scFv-Fc antibodies to the CD300c protein with those of the JEWR-195 antibody.
FIG. 8 shows the amino acid sequences of seven types of anti-CD300c scFv-Fc antibodies.
FIG. 9 shows the results of SDS-PAGE and Coomassie brilliant blue staining of the JEWR-195 antibody under reducing and non-reducing conditions.
FIG. 10 shows the flow cytometric analysis confirming that the JEWR-195 antibody binds to CD300c on the cell surface in HEK293T cells overexpressing CD300c.
FIG. 11 shows the results of Western blot analysis of the action of the JEWR-195 antibody on the NF-κB signaling pathway of dimeric TCTP in bronchial epithelial cells.
FIG. 12 is a graph showing comparison of the binding strengths of four types of anti-CD300c scFv-Fc antibodies to the mouse CD300c protein, as determined by ELISA.
FIG. 13 shows the results of SDS-PAGE and Coomassie brilliant blue staining of the JEWR-195 antibody under reducing and non-reducing conditions.
FIG. 14 shows the method of constructing the asthma mouse model and the administration schedule of the JEWR-M10 antibody.
FIG. 15 shows images of cells in the bronchoalveolar lavage fluid (BALF) of mice stained with Diff-Quick, along with graphs showing the results of total cell count measurements.
FIG**.** 16a shows PAS staining images of mouse lung tissue.
FIG. 16b is a graph showing the levels of MUC5AC production in the mouse BALF, as measured by ELISA.
FIG. 17a is a graph showing the production levels of IL-4, IL-5, and IL-13 in mouse lung tissue, as measured by ELISA.
FIG. 17b is a graph showing the production levels of IL-4, IL-5, and IL-13 in the mouse BALF, as measured by ELISA.

### [Best Mode]

The present inventors have for the first time identified CD300c, present in the cell membrane, as a receptor for dimeric translationally controlled tumor protein (TCTP). Since the inhibition of CD300c suppresses its interaction with dimeric TCTP and thereby effectively inhibits the cytokine-like activity of dimeric TCTP, this approach, which targets various CD300c-expressing cells, is significantly useful for the treatment of dimeric TCTP-related diseases, including allergic and inflammatory diseases, as well as malaria.

Hereinafter, the configuration and effects of the present disclosure will be explained in more detail below through Examples. These Examples are for illustrative purposes only, and the scope of the present disclosure is not limited by these Examples.

### <Example 1> Expression and Purification of Recombinant Dimeric TCTP Protein

To produce dimeric TCTP protein, a pRSET A (Invitrogen) expression vector was constructed by inserting a gene (SEQ ID NO: 2) in which the N-terminal 10 amino acids of TCTP (SEQ ID NO: 1) were deleted. The vector was then transformed into E. coli BL21(DE3)pLysS (Merck), and the cells were cultured to induce recombinant protein synthesis.

The culture medium was centrifuged, and the resulting pellet was resuspended in a solution of 20 mM Tris-HCl, 300 mM NaCl, 5 mM Imidazole, and 1 mM PMSF (pH 8.0), and the cells were lysed using an ultrasonic disruptor. Subsequently, the supernatant obtained by centrifugation was purified using Ni-NTA Agarose (Qiagen). To obtain high-purity protein, the protein was further purified using a HiTrap Q HP anion exchange chromatography column (Cytiva). The column was washed with triple-distilled water and then stabilized by flowing 20 mM Tris-HCl, 50 mM NaCl, and 1 mM EDTA (pH 7.4) for approximately 20 minutes. The previously purified protein was loaded onto the column, and eluted at a flow rate of 0.8 ml/min with a 0 to 1 M NaCl concentration gradient. The eluted proteins were subjected to SDS-PAGE under non-reducing conditions and Coomassie Brilliant Blue staining, and fractions in which dimeric TCTP was identified were selected. Using a PD-10 column (Cytiva), the buffer was exchanged with phosphate buffered saline (PBS), and the protein was concentrated using Amicon Ultra Centrifugal Filters (Millipore).

### <Example 2> Confirmation of Interaction between Dimeric TCTP and CD300c Protein

To confirm the interaction between dimeric TCTP and CD300c protein, a pull-down assay was performed using Dynabeads Protein A (Invitrogen). For the analysis, a recombinant human CD300c-Fc chimeric protein (BioLegend) was used. This protein contained the sequence Met29-Arg183, which corresponds to the extracellular domain of CD300c (SEQ ID NO: 3), and hIgG Fc at the C-terminus, and was expressed in 293E cells. As a control, recombinant human IgG1 Fc protein (BioLegend) was used. Since human immunoglobulin (Ig) binds to protein A beads, the beads were incubated with the above proteins at room temperature for 30 minutes. The cultured proteins were washed three times with PBS containing 0.02% Tween-20, and the protein-bound beads were then incubated with recombinant dimeric or monomeric TCTP at room temperature for 1 hour. Unbound proteins were removed by three washes, and the proteins bound to the beads were eluted and analyzed by Western blotting. The analysis was performed under non-reducing conditions, and detection was carried out using an anti-TCTP antibody (Santa Cruz Biotechnology).

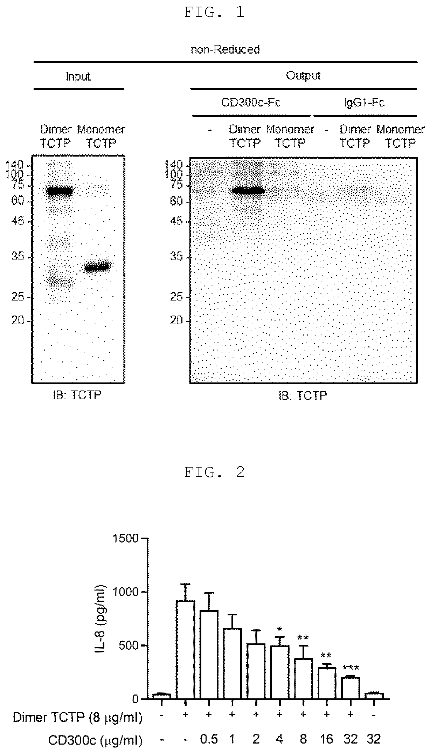

As shown in FIG. 1, dimeric TCTP was detected at a higher level than monomeric TCTP in beads bound to the CD300c-Fc protein, indicating an interaction between dimeric TCTP and the extracellular domain of CD300c. Little to no monomeric or dimeric TCTP was detected in the control group, beads bound to the IgG1-Fc protein.

### <Example 3> Confirmation of IL-8 Secretion Inhibitory Efficacy of CD300c Extracellular Domain Against Dimeric TCTP in Bronchial Epithelial Cells

Through the above results, the binding of dimeric TCTP and the CD300c protein was confirmed. Since BEAS-2B cells, a bronchial epithelial cell line, secrete the cytokine IL-8 when activated by dimeric TCTP, this was used to evaluate the inhibitory efficacy of the CD300c extracellular domain against dimeric TCTP. BEAS-2B cells were cultured in DMEM medium containing 10% FBS and 1% penicillin-streptomycin at 37°C in the presence of 5% carbon dioxide. Recombinant CD300c protein (Sino Biological) was expressed in HEK293 cells as the extracellular domain Met1-Arg183 of CD300c (SEQ ID No: 3). The CD300c extracellular domain (0-32 µg/ml) was pre-cultured with dimeric TCTP (8 µg/ml) at room temperature for 30 minutes, and then applied to the BEAS-2B cells and cultured for 20 hours. The levels of secreted IL-8 in the cell supernatant were quantified using an IL-8 ELISA kit (BioLegend).

As shown in FIG. 2, it was confirmed that the IL-8 secretion, which increased when BEAS-2B cells were treated with dimeric TCTP alone, was reduced in a concentration-dependent manner by pre-culturing with the CD300c extracellular domain. These results suggest that the CD300c extracellular domain suppresses intracellular activity by inhibiting the interaction between dimeric TCTP and CD300c on the cell membrane.

### <Example 4> Confirmation of Changes in CD300c Expression in Bronchial Epithelial Cells Following Treatment with Dimeric TCTP

To confirm changes in CD300c protein expression following treatment with dimeric TCTP, BEAS-2B cells were cultured in a 6-well plate and treated with dimeric TCTP (8 µg/ml) for 0.5, 1, 2, 4, 8, 20, and 24 hours, respectively. Then, the cells were washed with PBS and lysed in a buffer containing 50 mM Tris-HCl (pH 7.4), 150 mM NaCl, 1 mM EDTA (pH 8.0), 0.25% deoxycholate, 1% Triton X-100, a phosphatase inhibitor cocktail (Sigma-Aldrich), and a protease inhibitor (Roche). The supernatant obtained by centrifugation was analyzed for concentration using a Bradford assay, and Western blot analysis was performed using anti-CD300c antibodies (Abcam) and anti-GAPDH antibodies (Cell Signaling Technology).

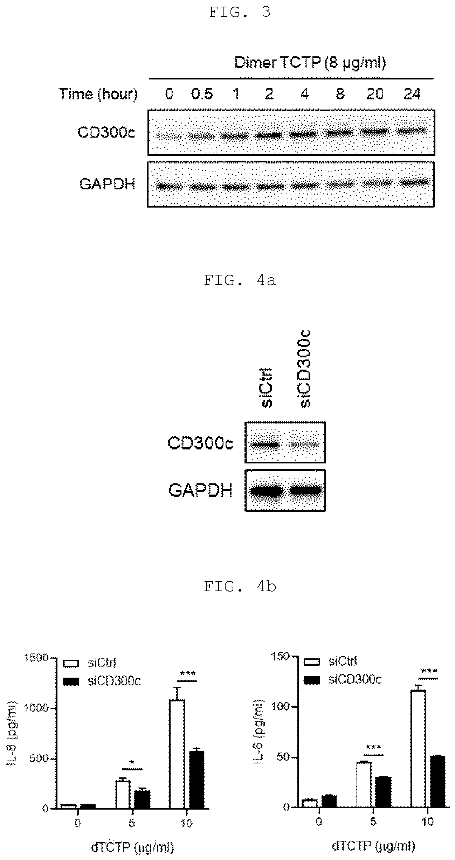

As shown in FIG. 3, it was confirmed that the expression of CD300c increased over time due to treatment with dimeric TCTP.

### <Example 5> Confirmation of Changes in Cytokine Secretion Induced by Dimeric TCTP Following CD300c Knockdown

To determine whether the inflammatory response induced by dimeric TCTP occurs via CD300c, CD300c was knocked down in BEAS-2B cells. CD300c siRNA (Thermo Fisher Scientific) or control siRNA (Santa Cruz Biotechnology) was transfected into cells using Lipofectamine RNAiMAX (Thermo Fisher Scientific) according to the manufacturer's recommended procedure. After 24 hours, the cells were treated with dimeric TCTP (0, 5, or 10 µg/ml) and cultured for 20 hours. The levels of IL-8 and IL-6 secreted in the cell supernatant were quantified using an ELISA kit.

As shown in FIG. 4a, CD300c expression decreased by 50% following CD300c knockdown in BEAS-2B cells.

As shown in FIG. 4b, it was confirmed that the secretion of IL-8 and IL-6, which increased due to treatment with dimeric TCTP in the control group siCtrl, was significantly reduced due to the knockdown of CD300c. These results demonstrate that dimeric TCTP induces an inflammatory response via the CD300c receptor.

### <Example 6> Confirmation of NF-κB Activity Induced by Dimeric TCTP Following CD300c Overexpression

To investigate the regulation of the cellular signaling pathway of dimeric TCTP by the overexpression of CD300c, HEK293 cell lines expressing an NF-κB activity-dependent reporter gene were used. Cells were cultured in MEM medium (Cytiva) containing 10% FBS, 1% non-essential amino acids, 1 mM Na pyruvate, 1% penicillin/streptomycin, and 50 µg/ml hygromycin B at 37°C in the presence of 5% carbon dioxide. The pCMV6-CD300c vector (ORIGENE) containing CD300c (SEQ ID NO: 4) was transfected into the cells using Lipofectamine 2000 (Thermo Fisher Scientific) according to the manufacturer's recommended procedure, and after 24 hours, the cells were cultured in medium supplemented with 400 µg/ml of G418. Selected cells were cultured in 96-well white plates and treated with dimeric TCTP (0, 8, or 16 µg/ml) for 6 hours. Luciferase activity was then measured using the ONE-Glo^{™} Luciferase Assay System (Promega) according to the manufacturer's recommended procedure.

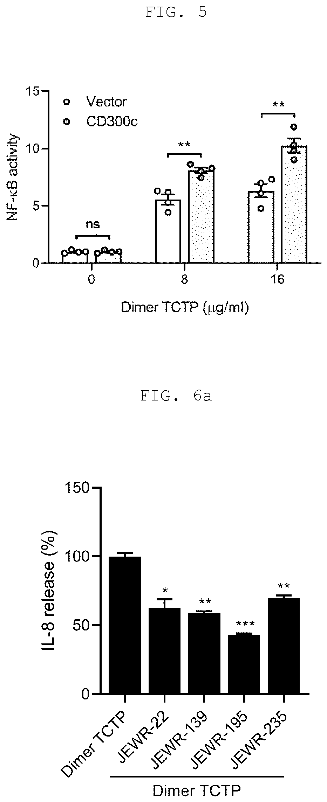

As shown in FIG. 5, in CD300c-overexpressing cells, treatment with dimeric TCTP led to a significant, dose-dependent increase in NF-κB activity. These results demonstrate that dimeric TCTP activates the NF-κB signaling pathway through CD300c.

### <Example 7> Preparation of Anti-CD300c Monoclonal Antibodies

### 7-1. Screening of Anti-CD300c Monoclonal Antibodies

Single-chain variable fragment (scFv) clones were isolated from a human scFv phage library via a panning process using the recombinant CD300c protein (Sino Biological), which is the human CD300c extracellular domain Met1-Arg183. CD300c extracellular domain antigen (5 µg) was adsorbed onto the surface of an immunoassay tube. The following day, the tubes were washed three times with phosphate buffered saline-Tween20 (PBST), blocked with 3% skim milk at room temperature for 1 hour, and then a 10¹³ pfu antibody phage library was added to react with the antigen. After washing with PBST, antigen-specifically bound phages were recovered by adding 100 mM TEA and allowing the solution to stand. The resulting solution was neutralized with 1 M Tris-HCl (pH 7.4), followed by E. coli infection to quantify the recovered phages, which were subsequently stored in SB culture medium. The phage solution from the previous cycle was taken and re-inoculated into E. coli, followed by phage amplification by adding helper phages. Antibody candidates specifically binding to the CD300c antigen was secured by repeating the panning process three times. To identify clones binding to the antigen, antigens at a concentration of 1 µg/ml were coated onto 96-well plates with sodium bicarbonate buffer (pH 9.6). The plates were washed three times with PBST and blocked with 3% skim milk for 1 hour. Subsequently, the obtained peripheral cytoplasmic extract was added and reacted at room temperature for 1 hour to bind to the antigen. The secondary antibody for detection was diluted 1 : 2,000 and incubated for 1 hour, and the reaction was carried out by adding TMB substrate. After 5 minutes, the reaction was stopped with 1 N HCl, and positive clones were selected by measuring the absorbance at 450 nm. Based on the above results, 148 clones showing a positive reaction to CD300c were selected from 480 clones. The selected positive clones were subjected to DNA sequencing to analyze the base sequences of the clones. As a result, 20 types of anti-CD300c monoclonal antibodies with different amino acid sequences were obtained.

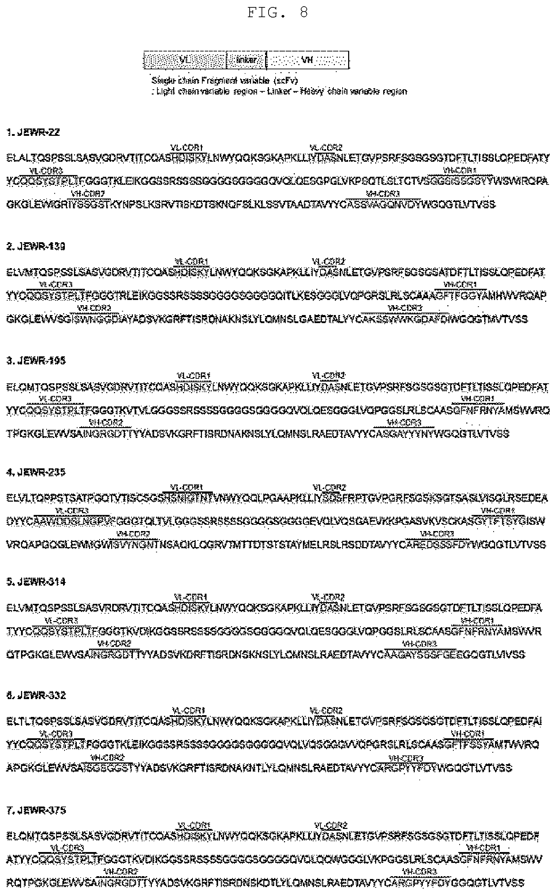

Seven anti-CD300c scFv antibodies with excellent efficacy were identified through cell experiments, and sequences thereof are shown in FIG. 8.

In addition, the DNA and amino acid sequences of the seven anti-CD300c scFv antibodies are shown in Table 1.

**[Table 1]**

| Antibo dy | | Sequence | | | |
|---|---|---|---|---|---|
| **[151]** J EWR-22 | | DNA Sequenc e | | GAGCTCGCACTCACGCAGTCTCCATCCTCCCTGTCTGCTTCTGTA GGAGACAGAGTCACCATCACTTGCCAAGCGAGTCACGACATTAGC AAATATTTAAATTGGTATCAACAGAAATCAGGGAAAGCCCCTAAG CTCCTGATCTACGATGCGTCCAATTTGGAAACAGGAGTCCCGTCA AGGTTCAGTGGCAGTGGATCTGGGACAGATTTCACTCTCACCATC AGCAGTCTGCAACCTGAAGATTTTGCAACTTACTACTGTCAACAG AGTTACAGTACCCCGCTCACTTTCGGCGGAGGGACCAAGCTGGAG ATCAAAGGTGGTTCCTCTAGATCTTCCTCCTCTGGTGGCGGTGGC TCGGGCGGTGGTGGGCAGGTGCAGCTGCAGGAGTCGGGCCCAGGA CTGGTGAAGCCTTCACAGACCCTGTCCCTCACCTGCACTGTCTCT GGTGGCTCCATCAGCAGTGGTAGTTACTACTGGAGCTGGATCCGG CAGCCCGCCGGGAAGGGACTGGAGTGGATTGGGCGTATTTATAGT AGTGGGAGCACCAAGTACAACCCCTCCCTCAAGAGCCGAGTCACC ATATCAAAGGACACGTCCAAGAATCAGTTCTCCCTGAAGCTGAGC TCTGTGACCGCCGCGGACACGGCCGTGTATTACTGTGCGAGTTCA GTGGCTGGACAGAACGTCGACTACTGGGGCCAGGGAACCCTGGTC ACCGTCTCCTCA (SEQ ID NO: 5) | |
| | | Am ino ac | Ful l len gth | ELALTQSPSSLSASVGDRVTITCQASHDISKYLNWYQQKSGKAPK LLIYDASNLETGVPSRFSGSGSGTDFTLTISSLQPEDFATYYCQQ SYSTPLTFGGGTKLEIKGGSSRSSSSGGGGSGGGGQVQLQESGPG LVKPSQTLSLTCTVSGGSISSGSYYWSWIRQPAGKGLEWIGRIYS | |
| | id Se qu en ce | | SGSTKYNPSLKSRVTISKDTSKNQFSLKLSSVTAADTAVYYCASS VAGQNVDYWGQGTLVTVSS (SEQ ID NO: 6) | | |
| | | Var iab le reg ion | Light Chain Variable Region | | |
| | | | TQSPSSLSASVGDRVTITCQASHDISKYLNWYQQKSGKAPKLLIY DASNLETGVPSRFSGSGSGTDFTLTISSLQPEDFATYYCQQSYST PLTFGGGTKLEIK (SEQ ID NO: 7) | | |
| | | | Heavy Chain Variable Region | | |
| | | | QVQLQESGPGLVKPSQTLSLTCTVSGGSISSGSYYWSWIRQPAGK GLEWIGRIYSSGSTKYNPSLKSRVTISKDTSKNQFSLKLSSVTAA DTAVYYCASSVAGQNVDYWGQGTLVTVSS (SEQ ID NO: 8) | | |
| | | CDR | Light Chain CDR1 | Light Chain CDR2 | Light Chain CDR3 |
| | | | HDISKY (SEQ ID NO: 9) | DAS (SEQ ID NO: 10) | QQSYSTPLT (SEQ ID NO: 11) |
| | | | Heavy Chain CDR1 | Heavy Chain CDR2 | Heavy Chain CDR3 |
| | | | GGSISSGSYY (SEQ ID NO: 12) | IYSSGST (SEQ ID NO: 13) | ASSVAGQNVDY (SEQ ID NO: 14) |
| | | Lin ker | GGSSRSSSSGGGGSGGGG (SEQ ID NO: 15) | | |
| **[152]** J EWR-139 | DNA Sequenc e | | GAGCTCGTGATGACTCAGTCTCCATCCTCCCTGTCTGCTTCTGTA GGAGACAGAGTCACCATCACTTGCCAAGCGAGTCACGACATTAGC AAATATTTAAATTGGTATCAACAGAAATCAGGGAAAGCCCCTAAG CTCCTGATCTACGATGCGTCCAATTTGGAAACAGGAGTCCCGTCA AGGTTCAGTGGCAGTGGATCTGCGACAGATTTCACTCTCACCATC AGCAGTCTGCAACCTGAAGATTTTGCAACTTACTACTGTCAACAG AGTTACAGTACCCCGCTCACTTTCGGCGGAGGGACACGACTGGAG ATTAAAGGTGGTTCCTCTAGATCTTCCTCCTCTGGTGGCGGTGGC TCGGGCGGTGGTGGGCAGATCACCTTGAAGGAGTCTGGGGGAGGC TTGGTACAGCCTGGCAGGTCCCTGAGACTCTCCTGTGCAGCCGCT GGATTCACCTTTGGTGGTTATGCCATGCACTGGGTCCGGCAAGCT CCAGGGAAGGGCCTGGAGTGGGTCTCAGGTATTAGTTGGAATGGT GGTGATATAGCCTATGCGGACTCTGTGAAGGGCCGATTCACCATC TCCAGAGACAACGCCAAGAACTCCCTGTATCTGCAAATGAACAGT CTGGGAGCTGAGGACACGGCCTTGTATTACTGTGCAAAATCTAGC TGGTGGAAGGGAGATGCCTTCGATATCTGGGGCCAAGGGACAATG GTCACCGTCTCCTCA (SEQ ID NO: 16) | | |
| | A m i n o a c i d | Full leng th | ELVMTQSPSSLSASVGDRVTITCQASHDISKYLNWYQQKSGKAPK LLIYDASNLETGVPSRFSGSGSATDFTLTISSLQPEDFATYYCQQ SYSTPLTFGGGTRLEIKGGSSRSSSSGGGGSGGGGQITLKESGGG LVQPGRSLRLSCAAAGFTFGGYAMHWVRQAPGKGLEWVSGISWNG GDIAYADSVKGRFTISRDNAKNSLYLQMNSLGAEDTALYYCAKSS WWKGDAFDIWGQGTMVTVSS (SEQ ID NO: 17) | | |
| | | Vari able Regi | Light Chain Variable Region | | |
| | | | MTQSPSSLSASVGDRVTITCQASHDISKYLNWYQQKSGKAPKLLI YDASNLETGVPSRFSGSGSATDFTLTISSLQPEDFATYYCQQSYS | | |
| | S e q u e n c e | on | TPLTFGGGTRLEIK SEQ ID NO: 18) | | |
| | | | Heavy Chain Variable Region | | |
| | | | QITLKESGGGLVQPGRSLRLSCAAAGFTFGGYAMHWVRQAPGKGL EWVSGISWNGGDIAYADSVKGRFTISRDNAKNSLYLQMNSLGAED TALYYCAKSSWWKGDAFDIWGQGTMVTVSS (SEQ ID NO: 19) | | |
| | | CDR | Light Chain CDR1 | Light Chain CDR2 | Light Chain CDR3 |
| | | | HDISKY (SEQ ID NO: 9) | DAS (SEQ ID NO: 10) | QQSYSTPLT (SEQ ID NO: 11) |
| | | | Heavy Chain CDR1 | Heavy Chain CDR2 | Heavy Chain CDR3 |
| | | | GFTFGGYA (SEQ ID NO: 20) | ISWNGGDI (SEQ ID NO: 21) | AKSSWWKGDAFDI (SEQ ID NO: 22) |
| | | Link er | GGSSRSSSSGGGGSGGGG (SEQ ID NO: 15) | | |
| **[153]** J EWR-195 | DNA Sequenc e | | GAGCTCCAGATGACCCAGTCTCCATCCTCCCTGTCTGCTTCTGTA GGAGACAGAGTCACCATCACTTGCCAAGCAAGTCACGACATTAGC AAATATTTAAATTGGTATCAACAGAAATCAGGGAAAGCCCCTAAG CTCCTGATCTACGATGCGTCCAATTTGGAAACAGGAGTCCCGTCA AGGTTCAGTGGCAGTGGATCTGGGACAGATTTCACTCTCACCATC AGCAGTCTGCAACCTGAAGATTTTGCAACTTACTACTGTCAACAG AGTTACAGTACCCCGCTCACTTTCGGCGGAGGGACCAAGGTGACC GTCCTAGGCGGTGGTTCCTCTAGATCTTCCTCCTCTGGTGGCGGT GGCTCGGGCGGTGGTGGGCAGGTGCAGCTGCAGGAGTCGGGGGGA GGCTTGGTACAGCCTGGGGGGTCCCTGAGACTCTCCTGTGCAGCC TCTGGATTCAACTTTAGGAATTATGCCATGAGCTGGGTCCGCCAG ACTCCAGGGAAGGGGCTGGAGTGGGTCTCAGCTATTAATGGTAGA GGTGATACCACATACTACGCAGACTCCGTGAAGGGCCGATTCACC ATCTCCAGAGACAATGCCAAGAACTCACTGTATCTGCAAATGAAC AGCCTGAGAGCCGAGGACACGGCTGTATACTACTGTGCAAGTGGC GCCTACTATTACAACTACTGGGGCCAGGGAACCCTGGTCACCGTC TCCTCA (SEQ ID NO: 23) | | |
| | A m i n o a c i d S e q | Full leng th | ELQMTQSPSSLSASVGDRVTITCQASHDISKYLNWYQQKSGKAPK LLIYDASNLETGVPSRFSGSGSGTDFTLTISSLQPEDFATYYCQQ SYSTPLTFGGGTKVTVLGGGSSRSSSSGGGGSGGGGQVQLQESGG GLVQPGGSLRLSCAASGFNFRNYAMSWVRQTPGKGLEWVSAINGR GDTTYYADSVKGRFTISRDNAKNSLYLQMNSLRAEDTAVYYCASG AYYYNYWGQGTLVTVSS (SEQ ID NO: 24) | | |
| | | Vari able Regi on | Light Chain Variable Region | | |
| | | | QMTQSPSSLSASVGDRVTITCQASHDISKYLNWYQQKSGKAPKLL IYDASNLETGVPSRFSGSGSGTDFTLTISSLQPEDFATYYCQQSY STPLTFGGGTKVTVLG (SEQ ID NO: 25) | | |
| | | | Heavy Chain Variable Region | | |
| | u e n c e | | QVQLQESGGGLVQPGGSLRLSCAASGFNFRNYAMSWVRQTPGKGL EWVSAINGRGDTTYYADSVKGRFTISRDNAKNSLYLQMNSLRAED TAVYYCASGAYYYNYWGQGTLVTVSS (SEQ ID NO: 26) | | |
| | | CDR | Light Chain CDR1 | Light Chain CDR2 | Light Chain CDR3 |
| | | | HDISKY (SEQ ID NO: 9) | DAS (SEQ ID NO: 10) | QQSYSTPLT (SEQ ID NO: 11) |
| | | | Heavy Chain CDR1 | Heavy Chain CDR2 | Heavy Chain CDR3 |
| | | | GFNFRNYA (SEQ ID NO: 27) | INGRGDTT (SEQ ID NO: 28) | ASGAYYYNY (SEQ ID NO: 29) |
| | | Link er Link er | GGSSRSSSSGGGGSGGGG (SEQ ID NO: 15) | | |
| **[154]** J EWR-235 | DNA Sequenc e | | GAGCTCGTGCTGACTCAGCCACCCTCAACGTCTGCGACCCCCGGG CAGACGGTCACCATCTCCTGTTCTGGAAGCCACTCCAACATCGGG ACTAATACTGTGAACTGGTACCAGCAACTCCCAGGGGCGGCCCCC AAGCTCCTCATCTACAGTGATTCTTTTCGACCCACTGGGGTCCCT GGCCGATTCTCTGGCTCCAAGTCTGGCACCTCGGCCTCCCTGGTC ATCAGTGGGCTCCGGTCCGAGGATGAGGCTGATTATTACTGTGCA GCATGGGATGACAGCCTGAATGGTCCGGTGTTCGGCGGAGGGACC CAGCTGACCGTCCTCGGCGGTGGTTCCTCTAGATCTTCCTCCTCT GGTGGCGGTGGCTCGGGCGGTGGTGGGGAGGTGCAGCTGGTGCAG TCTGGAGCTGAGGTGAAGAAGCCTGGGGCCTCAGTAAAGGTCTCC TGCAAGGCTTCTGGTTACACCTTTACCAGTTATGGTATCAGCTGG GTGCGACAGGCCCCTGGACAAGGGCTTGAGTGGATGGGATGGATC AGTGTTTACAATGGTAACACAAACTCCGCACAGAAGCTCCAGGGC AGAGTCACCATGACCACAGACACATCCACGAGCACAGCCTACATG GAGCTGAGGAGCCTGAGATCTGACGACACGGCCGTGTATTACTGT GCGAGAGAGGATAGCAGCTCGTTTGACTACTGGGGCCAGGGAACC CTGGTCACCGTCTCCTCA (SEQ ID NO: 30) | | |
| | A m i n o a c i d S e q u e n c e | Full leng th | ELVLTQPPSTSATPGQTVTISCSGSHSNIGTNTVNWYQQLPGAAP KLLIYSDSFRPTGVPGRFSGSKSGTSASLVISGLRSEDEADYYCA AWDDSLNGPVFGGGTQLTVLGGGSSRSSSSGGGGSGGGGEVQLVQ SGAEVKKPGASVKVSCKASGYTFTSYGISWVRQAPGQGLEWMGWI SVYNGNTNSAQKLQGRVTMTTDTSTSTAYMELRSLRSDDTAVYYC AREDSSSFDYWGQGTLVTVSS (SEQ ID NO: 31) | | |
| | | Vari able Regi on | Light Chain Variable Region | | |
| | | | VLTQPPSTSATPGQTVTISCSGSHSNIGTNTVNWYQQLPGAAPKL LIYSDSFRPTGVPGRFSGSKSGTSASLVISGLRSEDEADYYCAAW DDSLNGPVFGGGTQLTVLG (SEQ ID NO: 32) | | |
| | | | Heavy Chain Variable Region | | |
| | | | EVQLVQSGAEVKKPGASVKVSCKASGYTFTSYGISWVRQAPGQGL EWMGWISVYNGNTNSAQKLQGRVTMTTDTSTSTAYMELRSLRSDD TAVYYCAREDSSSFDYWGQGTLVTVSS (SEQ ID NO: 33) | | |
| | | CDR | Light Chain CDR1 | Light Chain CDR2 | Light Chain CDR3 |
| | | | HSNIGTNT (SEQ ID NO: 34) | SDS (SEQ ID NO: 35) | AAWDDSLNGPV (SEQ ID NO: 36) |
| | | | Heavy Chain CDR1 | Heavy Chain CDR2 | Heavy Chain CDR3 |
| | | | GYTFTSYG (SEQ ID NO: 37) | ISVYNGNT (SEQ ID NO: 38) | AREDSSSFDY (SEQ ID NO: 39) |
| | | Link er | GGSSRSSSSGGGGSGGGG (SEQ ID NO: 15) | | |
| **[155]** J EWR-314 | DNA Sequenc e | | GAGCTCGTGATGACTCAGTCTCCATCCTCCCTGTCTGCTTCTGTA AGAGACAGAGTCACCATCACTTGCCAAGCGAGTCACGACATTAGC AAATATTTAAATTGGTATCAACAGAAATCAGGGAAAGCCCCTAAG CTCCTGATCTACGATGCGTCCAATTTGGAAACAGGAGTCCCGTCA AGGTTCAGTGGCAGTGGATCTGGGACAGATTTCACTCTCACCATC AGCAGTCTGCAACCTGAAGATTTTGCAACTTACTACTGTCAACAG AGTTACAGTACCCCGCTCACTTTCGGCGGAGGGACCAAAGTGGAT ATCAAAGGTGGTTCCTCTAGATCTTCCTCCTCTGGTGGCGGTGGC TCGGGCGGTGGTGGGCAGGTGCAGCTGCAGGAGTCGGGGGGAGGC TTGGTACAGCCTGGGGGGTCCCTGAGACTCTCCTGTGCAGCCTCT GGATTCAACTTTAGGAATTATGCCATGAGCTGGGTCCGCCAGACT CCAGGGAAGGGGCTGGAGTGGGTCTCAGCTATTAATGGTAGAGGT GATACCACATACTACGCAGACTCCGTGAAGGACCGATTCACCATC TCCAGAGACAATTCCAAGAACTCACTGTATCTACAAATGAACAGC CTGAGAGCCGAGGACACGGCCGTGTATTACTGTGCGGCCGGTGCT TATAGCAGCTCGTTTGGCGAAGAGGGCCAGGGAACCCTGGTCATC GTCTCCTCA (SEQ ID NO: 40) | | |
| | A m i n o a c i d S e q u e n c e | Full leng th | ELVMTQSPSSLSASVRDRVTITCQASHDISKYLNWYQQKSGKAPK LLIYDASNLETGVPSRFSGSGSGTDFTLTISSLQPEDFATYYCQQ SYSTPLTFGGGTKVDIKGGSSRSSSSGGGGSGGGGQVQLQESGGG LVQPGGSLRLSCAASGFNFRNYAMSWVRQTPGKGLEWVSAINGRG DTTYYADSVKDRFTISRDNSKNSLYLQMNSLRAEDTAVYYCAAGA YSSSFGEEGQGTLVIVSS (SEQ ID NO: 41) | | |
| | | Vari able Regi on | Light Chain Variable Region | | |
| | | | MTQSPSSLSASVRDRVTITCQASHDISKYLNWYQQKSGKAPKLLI YDASNLETGVPSRFSGSGSGTDFTLTISSLQPEDFATYYCQQSYS TPLTFGGGTKVDIK (SEQ ID NO: 42) | | |
| | | | Heavy Chain Variable Region | | |
| | | | QVQLQESGGGLVQPGGSLRLSCAASGFNFRNYAMSWVRQTPGKGL EWVSAINGRGDTTYYADSVKDRFTISRDNSKNSLYLQMNSLRAED TAVYYCAAGAYSSSFGEEGQGTLVIVSS (SEQ ID NO: 43) | | |
| | | CDR | Light Chain CDR1 | Light Chain CDR2 | Light Chain CDR3 |
| | | | HDISKY (SEQ ID NO: 9) | DAS (SEQ ID NO: 10) | QQSYSTPLT (SEQ ID NO: 11) |
| | | | Heavy Chain CDR1 | Heavy Chain CDR2 | Heavy Chain CDR3 |
| | | | GFNFRNYA (SEQ ID NO: 27) | INGRGDTT (SEQ ID NO: 28) | AAGAYSSSFGE (SEQ ID NO: 44) |
| | | Link er | GGSSRSSSSGGGGSGGGG (SEQ ID NO: 15) | | |
| | DNA Sequenc e | | GAGCTCACACTCACGCAGTCTCCATCCTCCCTGTCTGCTTCTGTA GGAGACAGAGTCACCATCACTTGCCAAGCGAGTCACGACATTAGC AAATATTTAAATTGGTATCAACAGAAATCAGGGAAAGCCCCTAAG CTCCTGATCTACGATGCGTCCAATTTGGAAACAGGAGTCCCGTCA AGGTTCAGTGGCAGTGGATCTGGGACAGATTTCACTCTCACCATC AGCAGTCTGCAACCTGAAGATTTTGCAATTTACTACTGTCAACAG AGTTACAGTACCCCGCTCACTTTCGGCGGAGGGACCAAGCTGGAG ATCAAAGGTGGTTCCTCTAGATCTTCCTCCTCTGGTGGCGGTGGC TCGGGCGGTGGTGGGCAGGTGCAGCTGGTGCAGTCTGGGGGAGGC GTGGTCCAGCCTGGGAGGTCCCTGAGACTCTCCTGTGCAGCCTCT GGATTCACCTTCAGTAGCTATGCCATGACCTGGGTCCGCCAGGCT CCAGGGAAGGGGCTGGAGTGGGTCTCAGCTATTAGTGGTAGTGGT GGTAGCACATACTACGCAGACTCCGTGAAGGGCCGGTTCACCATC TCCAGAGACAACGCCAAGAACACGCTGTATCTGCAAATGAACAGT CTGAGAGCCGAGGACACGGCTGTGTATTACTGTGCAAGAGGCCCG TACTACTTTGACTACTGGGGCCAGGGAACCCTGGTCACCGTCTCC TCA (SEQ ID NO: 45) | | |
| **[156]** J EWR-332 | A m i n o a c i d S e q u e n c e | Full leng th | ELTLTQSPSSLSASVGDRVTITCQASHDISKYLNWYQQKSGKAPK LLIYDASNLETGVPSRFSGSGSGTDFTLTISSLQPEDFAIYYCQQ SYSTPLTFGGGTKLEIKGGSSRSSSSGGGGSGGGGQVQLVQSGGG VVQPGRSLRLSCAASGFTFSSYAMTWVRQAPGKGLEWVSAISGSG GSTYYADSVKGRFTISRDNAKNTLYLQMNSLRAEDTAVYYCARGP YYFDYWGQGTLVTVSS (SEQ ID NO: 46) | | |
| | | Vari able Regi on | Light Chain Variable Region | | |
| | | | TQSPSSLSASVGDRVTITCQASHDISKYLNWYQQKSGKAPKLLIY DASNLETGVPSRFSGSGSGTDFTLTISSLQPEDFAIYYCQQSYST PLTFGGGTKLEIK (SEQ ID NO: 47) | | |
| | | | Heavy Chain Variable Region | | |
| | | | QVQLVQSGGGVVQPGRSLRLSCAASGFTFSSYAMTWVRQAPGKGL EWVSAISGSGGSTYYADSVKGRFTISRDNAKNTLYLQMNSLRAED TAVYYCARGPYYFDYWGQGTLVTVSS (SEQ ID NO: 48) | | |
| | | CDR | Light Chain CDR1 | Light Chain CDR2 | Light Chain CDR3 |
| | | | HDISKY (SEQ ID NO: 9) | DAS (SEQ ID NO: 10) | QQSYSTPLT (SEQ ID NO: 11) |
| | | | Heavy Chain CDR1 | Heavy Chain CDR2 | Heavy Chain CDR3 |
| | | | GFTFSSYA (SEQ ID NO: 49) | ISGSGGST (SEQ ID NO: 50) | ARGPYYFDY (SEQ ID NO: 51) |
| | | Link er | GGSSRSSSSGGGGSGGGG (SEQ ID NO: 15) | | |
| | DNA Sequenc e | | GAGCTCCAGATGACCCAGTCTCCATCCTCCCTGTCTGCTTCTGTA GGAGACAGAGTCACCATCACTTGCCAAGCGAGTCACGACATTAGC AAATATTTAAATTGGTATCAACAGAAATCAGGGAAAGCCCCTAAG CTCCTGATCTACGATGCGTCCAATTTGGAAACAGGAGTCCCGTCA AGGTTCAGTGGCAGTGGATCTGGGACAGATTTCACTCTCACCATC AGCAGTCTGCAACCTGAAGATTTTGCAACTTACTACTGTCAACAG AGTTACAGTACCCCGCTCACTTTCGGCGGAGGGACCAAAGTGGAT ATCAAAGGTGGTTCCTCTAGATCTTCCTCCTCTGGTGGCGGTGGC TCGGGCGGTGGTGGGCAGGTGCAGCTACAGCAGTGGGGGGGAGGC CTGGTCAAGCCTGGGGGGTCCCTGAGACTCTCCTGTGCAGCCTCT GGATTCAACTTTAGGAATTATGCCATGAGCTGGGTCCGCCAGACT CCAGGGAAGGGGCTGGAGTGGGTCTCAGCTATTAATGGTAGAGGT GATACCACATACTACGCAGACTCCGTGAAGGGCCGATTCACCATC TCCAGAGACAATTCCAAGGACACACTGTATCTGCAAATGAACAGT CTGAGAGCCGAGGACACGGCTGTGTATTACTGTGCAAGAGGCCCG TACTACTTTGACTACGGGGGCCAGGGAACCCTGGTCACCGTCTCC TCA (SEQ ID NO: 52) | | |
| JEWR-375 | A m i n o a c i d S e q u e n c e | Full leng th | ELQMTQSPSSLSASVGDRVTITCQASHDISKYLNWYQQKSGKAPK LLIYDASNLETGVPSRFSGSGSGTDFTLTISSLQPEDFATYYCQQ SYSTPLTFGGGTKVDIKGGSSRSSSSGGGGSGGGGQVQLQQWGGG LVKPGGSLRLSCAASGFNFRNYAMSWVRQTPGKGLEWVSAINGRG DTTYYADSVKGRFTISRDNSKDTLYLQMNSLRAEDTAVYYCARGP YYFDYGGQGTLVTVSS (SEQ ID NO: 53) | | |
| | | Vari able Regi on | Light Chain Variable Region | | |
| | | | QMTQSPSSLSASVGDRVTITCQASHDISKYLNWYQQKSGKAPKLL IYDASNLETGVPSRFSGSGSGTDFTLTISSLQPEDFATYYCQQSY STPLTFGGGTKVDIK (SEQ ID NO: 54) | | |
| | | | Heavy Chain Variable Region | | |
| | | | QVQLQQWGGGLVKPGGSLRLSCAASGFNFRNYAMSWVRQTPGKGL EWVSAINGRGDTTYYADSVKGRFTISRDNSKDTLYLQMNSLRAED TAVYYCARGPYYFDYGGQGTLVTVSS (SEQ ID NO: 55) | | |
| | | CDR | Light Chain CDR1 | Light Chain CDR2 | Light Chain CDR3 |
| | | | HDISKY (SEQ ID NO: 9) | DAS (SEQ ID NO: 10) | QQSYSTPLT (SEQ ID NO: 11) |
| | | | Heavy Chain CDR1 | Heavy Chain CDR2 | Heavy Chain CDR3 |
| | | | GFNFRNYA (SEQ ID NO: 27) | INGRGDTT (SEQ ID NO: 28) | ARGPYYFDY (SEQ ID NO: 51) |
| **[157]** | | Link er | GGSSRSSSSGGGGSGGGG (SEQ ID NO: 15) | | |

### 7-2. Preparation and Purification of Anti-CD300c Monoclonal Antibodies

To prepare anti-CD300c monoclonal antibodies, the genes were inserted into a vector containing a fusion of human scFv and IgG1, followed by transfection into 293F cells to induce expression. 50 ml of culture medium was centrifuged, and the obtained supernatant was purified using protein G agarose (GE) resin. Sequencing and production processes were performed, and 20 types of antibodies with different amino acid sequences were selected and purified. To confirm the purity of the antibodies, 2 µg of the antibodies were subjected to SDS-PAGE followed by Coomassie Brilliant Blue staining.

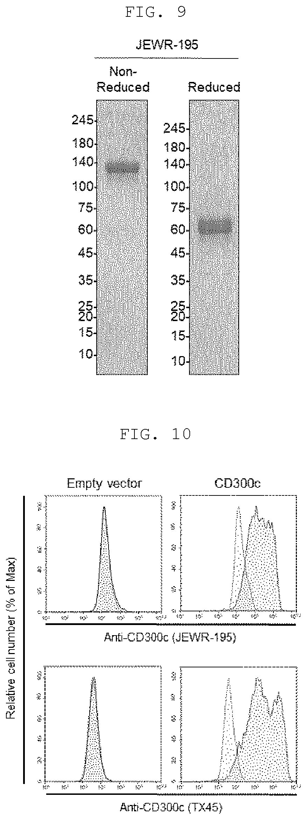

As illustrated in FIG. 9, the seven anti-CD300c scFv-Fc antibodies were produced and purified with a purity of 95% or more under both reducing and non-reducing conditions.

### 7-3. Confirmation of IL-8 Secretion Inhibitory Efficacy of Anti-CD300c Monoclonal Antibody Against Dimeric TCTP in Bronchial Epithelial Cells

BEAS-2B cells were used to confirm the efficacy of the monoclonal antibodies. BEAS-2B cells were pretreated with anti-CD300c monoclonal antibody (50 µg/ml) at 37°C for 30 minutes, and dimeric TCTP (4 µg/ml) was added, followed by incubation for 20 hours. The cell supernatant was collected, and the levels of secreted IL-8 were confirmed via ELISA.

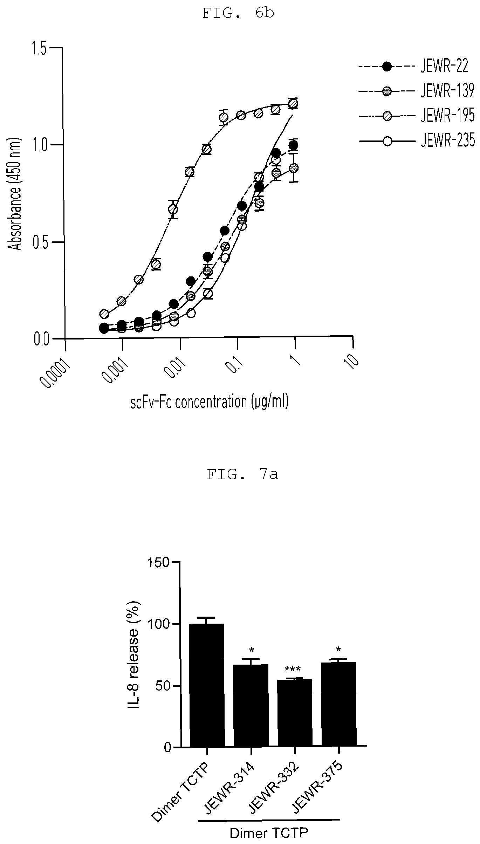

As shown in FIGS. 6a and 7a, primary screening identified four types of anti-CD300c monoclonal antibodies, JEWR-22, JEWR-139, JEWR-195, and JEWR-235 (FIG. 6a), and secondary screening identified three types of anti-CD300c monoclonal antibodies, JEWR-314, JEWR-332, and JEWR-375 (FIG. 7a), and all antibodies demonstrated the efficacy to inhibit dimeric TCTP-induced IL-8 secretion.

### 7-4. Confirmation of Antigen Binding Strengths of Anti-CD300c Monoclonal Antibodies

To select a monoclonal antibody with high antigen binding strength from the antibodies purified in Examples above, binding analysis was performed via ELISA. CD300c antigen at a concentration of 1 µg/ml was coated onto a 96-well plate with PBS. The following day, after washing three times with PBST, the plates were blocked with 1% skim milk at room temperature for 1 hour, and reacted with seven types of anti-CD300c antibodies at concentrations of 0 to 1 µg/ml at room temperature for 1 hour. Subsequently, HRP-conjugated anti-human IgG antibodies were diluted 1:20,000 and added for a reaction for 1 hour. Then, TMB substrate was added to induce color development, and the color reaction was terminated by adding 1 N HCl solution. The binding strengths of the antibodies were confirmed by measuring the absorbance at 450 nm, and the results for the seven types of anti-CD300c antibodies are shown in Table 2.

**[Table 2]**

| **Anti-CD300c antibody** | **EC₅₀ (ng/ml)** |
|---|---|
| JEWR-22 | 60.2 |
| JEWR-139 | 67.1 |
| JEWR-195 | 7.5 |
| JEWR-235 | 180.1 |
| JEWR-314 | 31 |
| JEWR-332 | 14.6 |
| JEWR-375 | 29.5 |

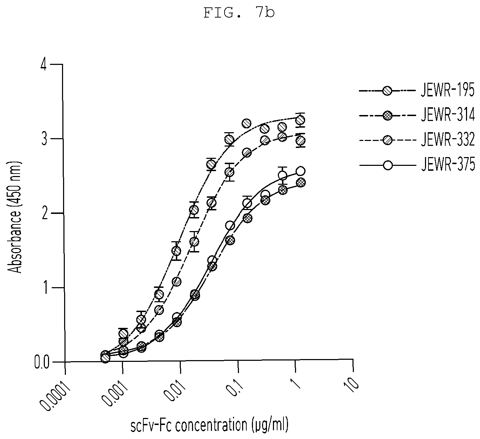

As shown in FIGS. 6b and 7b, the antigen-binding strength to CD300c was evaluated through the primary screening (FIG. 6b) in the order of JEWR-195 > JEWR-22 > JEWR-139 > JEWR-235, while the secondary screening (FIG. 7b) including the JEWR-195 antibody, which had the highest binding strength, showed that the binding strength was in the order of JEWR-195 > JEWR-332 > JEWR-375 > JEWR-314.

To confirm the binding strength of the monoclonal antibody to CD300c present on the cell membrane, the HEK293T cell line overexpressing CD300c was used. Cell lines overexpressing human CD300c were transfected with the pCMV6-CD300c vector using Lipofectamine 2000 according to the manufacturer's recommended procedure. Following 24 hours of culture, the cells were detached from the plate using accutase (Innovative Cell Technologies), washed with 1% BSA, and reacted with the anti-CD300c monoclonal antibody (10 µg/ml) at 4°C for 30 minutes. Subsequently, after washing to remove unbound antibodies, the cells were stained with a FITC-labeled anti-human IgG Fc secondary antibody (Invitrogen) at 4°C for 30 minutes, washed, and analyzed by flow cytometry.

As shown in FIG. 10, the overexpression of CD300c was confirmed using the PE anti-CD300c TX45 antibody (BD Biosciences), and the observed increase in fluorescence intensity confirmed that the JEWR-195 antibody binds to CD300c expressed on the cell membrane.

### <Example 8> Confirmation of Action of Anti-CD300c Monoclonal Antibody on NF-κB Signaling Pathway of Dimeric TCTP in Bronchial Epithelial Cells

Western blot was performed to confirm the mechanism of action of the JEWR-195 antibody, which exhibited excellent binding strength to the antigen and inhibitory effect on the activity of dimeric TCTP in Example above. BEAS-2B cells were cultured in 6-well plates, and pretreated with JEWR-195 antibody (0, 30, or 50 µg/ml) for 30 minutes, followed by treatment with dimeric TCTP (4 µg/ml), and the cells were cultured for 1 hour. The expression of phosphorylated IκBα (p-IκBα), IκBα, phosphorylated p65, p65, and GAPDH (cell signaling) was confirmed by Western blot.

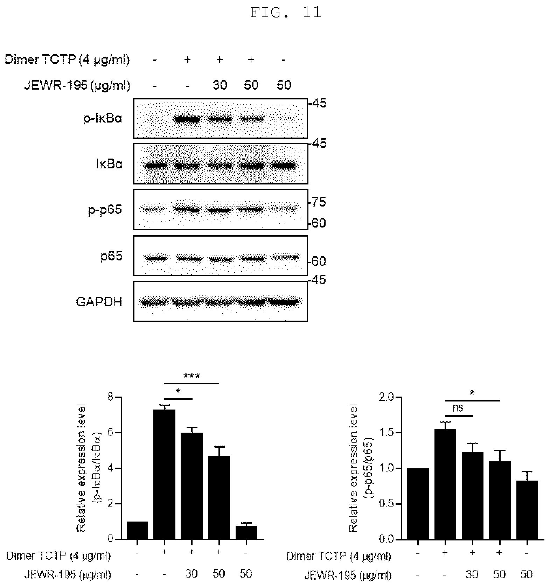

As shown in FIG. 11, the levels of p-IκBα and p-p65, which were increased by dimeric TCTP, were found to decrease in a concentration-dependent manner upon pretreatment with the JEWR-195 antibody.

Collectively, these results confirm that the anti-CD300c monoclonal antibody of the present disclosure inhibits NF-κB signaling induced by dimeric TCTP in bronchial epithelial cells and reduces the secretion of IL-8 cytokine, thereby exhibiting an effect of suppressing allergic reactions.

### <Example 9> Confirmation of Anti-CD300c Monoclonal Antibody's Anti-inflammatory Effect in Asthma Mouse Model

### 9-1. Screening of Anti-Mouse CD300c Monoclonal Antibodies

To confirm whether CD300c inhibition exerts anti-inflammatory efficacy in an asthma mouse model, a novel antibody that specifically binds to mouse CD300c was constructed.

A panning process was performed using a recombinant CD300c protein (MyBioSource) corresponding to the extracellular domain (His22-His188) of mouse CD300c (SEQ ID NO: 56), and scFv clones were isolated from a human scFv phage library. This process was performed in the same manner as in Example 7-1, and 14 clones showing positive reactivity to mouse CD300c were selected from a total of 288 clones. Subsequently, DNA sequencing of the selected clones was performed to analyze their nucleotide sequences, and as a result, four types of anti-mouse CD300c monoclonal antibodies with different amino acid sequences were obtained.

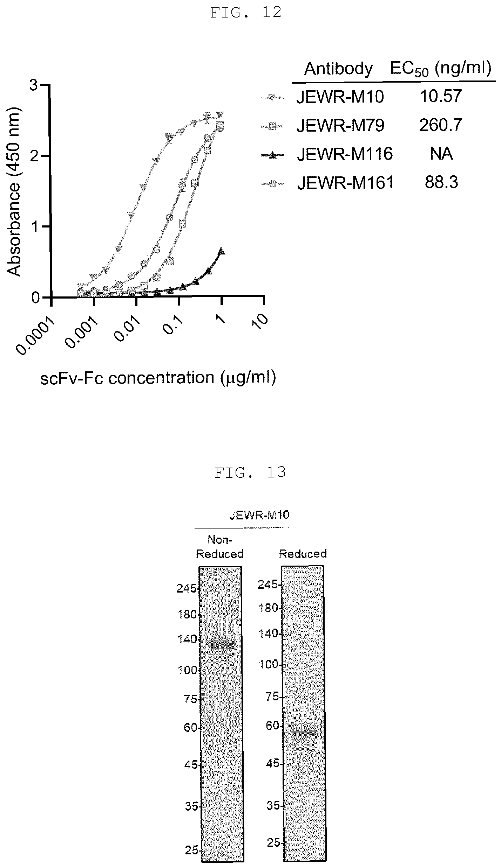

The four types of monoclonal antibodies obtained were prepared and purified in the same manner as in Example 7-2, and ELISA was performed in the same manner as in Example 7-4 to select a monoclonal antibody with high antigen binding strength from among the purified antibodies. As shown in FIG. 12, the binding strength to mouse CD300c was found to be in the order of JEWR-M10 > JEWR-M161 > JEWR-M79 > JEWR-M116.

In addition, as shown in FIG. 13, it was confirmed that the monoclonal antibody JEWR-M10, which exhibits excellent antigen binding strength, was purified to a purity of 95% or more in both reduced and non-reduced states. Table 3 shows the DNA sequence and amino acid sequence of JEWR-M10.

**[Table 3]**

| Antibo dy | | Sequence | | | |
|---|---|---|---|---|---|
| JEWR-M10 | | DNA Sequenc e | | GAGCTCGCCCTGACTCAGCCTCCCTCCGTGTCTGGGTCTCCTGGA CAGTCGATCACCCTCTCCTGCACTGGAACCAGCAGTGATATTGGT GATTATAACTATGTCTCCTGGTACCAACAACACCCAGGCAAAGCC CCCAAACTCATAATTTATGAGGTCAATAATCGGCCCTCAGGGGTT TCTATTCGCTTCTCTGGCTCCAAGTCTGGCAACACGGCCTCCCTG ACCATCTCTGGGCTCCAGGCTGAGGATGAGGCTGATTATTACTGC ACTTCATATACACCCAGGAGCATTGTCTTCGGCGGAGGGACCCAG | |
| | | | CTGACCGTCCTCGGTGGTGGTTCCTCTAGATCTTCCTCCTCTGGT GGCGGTGGCTCGGGCGGTGGTGGGGAGGTGCAGCTGGTGCAGTCT GGGGGAGGTGTGGTACGGCCTGGGGGGTCCCTGAGACTCTCCTGT GCAGCCTCTGGATTCACCTTTGATGATTATGGCATGAGCTGGGTC CGCCAAGCTCCAGGGAAGGGGCTGGAGTGGGTCTCTGGTATTAAT TGGAATGGTGGTAGCACAATTTATGCAGACTCTGTGAAGGGCCGA TTCATCATCTCCAGAGACAACGCCAAGAACTCCCTGTATCTGCAA ATGAACAGTCTGAGAGCCGAGGACACGGCCCTGTATTATTGTGTT CGTGGTCCCGACCTTGACTACTGGGGCCAGGGAACCCTGCTCATC GTCTCCTCA (SEQ ID NO: 57) | | |
| | Am in o ac id Se qu en ce | Ful l len gth | ELALTQPPSVSGSPGQSITLSCTGTSSDIGDYNYVSWYQQHPGKA PKLIIYEVNNRPSGVSIRFSGSKSGNTASLTISGLQAEDEADYYC TSYTPRSIVFGGGTQLTVLGGGSSRSSSSGGGGSGGGGEVQLVQS GGGVVRPGGSLRLSCAASGFTFDDYGMSWVRQAPGKGLEWVSGIN WNGGSTIYADSVKGRFIISRDNAKNSLYLQMNSLRAEDTALYYCV RGPDLDYWGQGTLLIVSS (SEQ ID NO: 58) | | |
| | | Var iab le Reg ion | Light Chain Variable Region | | |
| | | | ELALTQPPSVSGSPGQSITLSCTGTSSDIGDYNYVSWYQQHPGKA PKLIIYEVNNRPSGVSIRFSGSKSGNTASLTISGLQAEDEADYYC TSYTPRSIVFGGGTQLTVLG (SEQ ID NO: 59) | | |
| | | | Heavy Chain Variable Region | | |
| | | | EVQLVQSGGGVVRPGGSLRLSCAASGFTFDDYGMSWVRQAPGKGL EWVSGINWNGGSTIYADSVKGRFIISRDNAKNSLYLQMNSLRAED TALYYCVRGPDLDYWGQGTLLIVSS (SEQ ID NO: 60) | | |
| | | CDR | Light Chain CDR1 | Light Chain CDR2 | Light Chain CDR3 |
| | | | SSDIGDYNYVS (SEQ ID NO: 61) | EVNN (SEQ ID NO: 62) | TSYTPRS (SEQ ID NO: 63) |
| | | | Heavy Chain CDR1 | Heavy Chain CDR2 | Heavy Chain CDR3 |
| | | | DYGMS (SEQ ID NO: 64) | GINWNGGST (SEQ ID NO: 65) | GPDLDY (SEQ ID NO: 66) |
| | | Lin ker | GGSSRSSSSGGGGSGGGG (SEQ ID NO: 15) | | |

### 9-2. Construction of Asthma Mouse Model

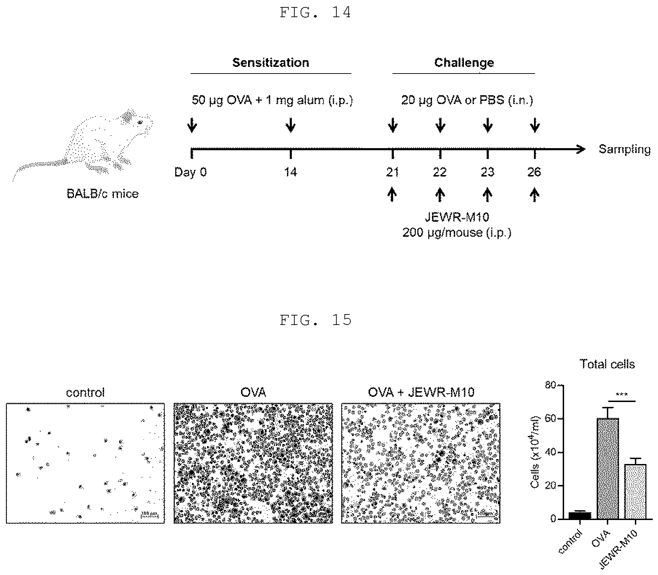

To confirm the anti-inflammatory effects of the anti-CD300c scFv-Fc antibody under in vivo conditions, an ovalbumin-induced asthma model was constructed using 6-week-old BALB/c mice as shown in FIG. 14. To induce allergic asthma, primary sensitization was performed by intraperitoneal injection of 50 µg ovalbumin (Thermo Scientific) and 1 mg alum (Invivogen), followed by secondary sensitization on Day 14 using the same method to induce an immune response. Subsequently, on Days 21, 22, 23, and 26, 20 µg of ovalbumin was administered intranasally to induce an allergic reaction challenge. The JEWR-M10 antibody was administered a total of four times, with an antibody dose of 200 µg/mouse. The antibody was administered via intraperitoneal (i.p.) injection 30 minutes prior to each intranasal (i.n.) challenge with ovalbumin. Forty-eight hours after the final antibody administration, mice were anesthetized with Zoletil^{®} (Virbac) and Rompun^{®} (Bayer) and sacrificed.

### 9-3. Confirmation of Anti-inflammatory Effects by Administration of Anti-CD300c Monoclonal Antibodies

In an asthma mouse model induced by repeated sensitization to ovalbumin, immune cells and inflammatory cytokines increase in bronchoalveolar lavage fluid and lung tissue. Accordingly, bronchoalveolar lavage fluid and lung tissue were collected to confirm the anti-inflammatory effects of JEWR-M10.

Mice were anesthetized and underwent thoracotomy. A 20-gauge endovascular catheter was inserted into the trachea, and 1 ml of PBS was instilled and aspirated three times to collect the bronchoalveolar lavage fluid (BALF). The recovered bronchoalveolar lavage fluid was centrifuged at 2000 rpm for 10 minutes, and the supernatant was subjected to ELISA for the measurement of MUC5AC and Th2 cytokines. The cell precipitate was resuspended in 100 µl of PBS to measure the total cell count, and the cells were plated onto a slide glass using Cytospin and stained with a Diff-Quick staining kit (Sysmex).

As shown in FIG. 15, the total cell count in the bronchoalveolar lavage fluid increased in the airway inflammation-induced group (OVA) compared to the control group, which decreased with the administration of JEWR-M10.

For the histological analysis of lung tissue, samples were fixed in a 4% formalin solution, dehydrated in ethanol, and prepared into paraffin blocks. These blocks were then sectioned to a thickness of 5 µm and stained with Periodic Acid-Schiff (PAS).

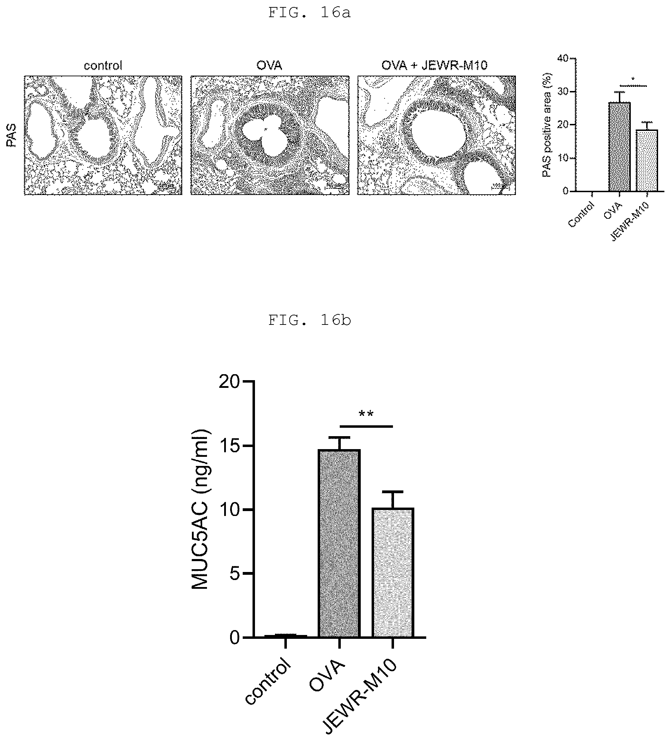

As shown in FIG. 16a, images obtained using a light microscope revealed an increase in peribronchial immune cell infiltration and in PAS-positive cells in the lung tissue of mice with airway inflammation induced by ovalbumin; however, the administration of JEWR-M10 was found to be alleviated these changes, resulting in a decrease in immune cell infiltration and goblet cell area.

Consistent with the above results, as shown in FIG. 16b, MUC5AC levels in the bronchoalveolar lavage fluid was significantly reduced in the antibody-treated group compared to the airway inflammation-induced group.

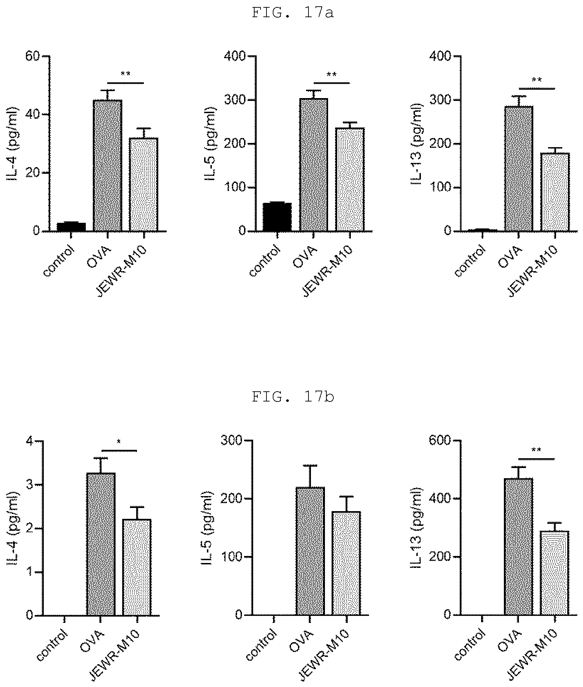

As shown in FIGS. 17a and 17b, the levels of Th2 cytokines IL-4, IL-5, and IL-13 increased in the airway inflammation-induced group, which significantly decreased in lung homogenates (FIG. 17a) and bronchoalveolar lavage fluid (FIG. 17b) upon administration of JEWR-M10.

These results demonstrate that the anti-CD300c monoclonal antibodies inhibit airway inflammatory responses by reducing immune cell infiltration, mucus secretion, and cytokine secretion in an asthma mouse model.

From the foregoing description, those skilled in the art to which the present disclosure pertains will understand that the present disclosure may be implemented in other specific forms without altering its technical concept or essential features. In this regard, the embodiments described above should be understood as being merely illustrative and not limiting in any respect. The scope of the present disclosure should be interpreted such that all modifications or variations derived from the meaning and scope of the appended claims and their equivalents, rather than from the above detailed description, are included within the scope of the present disclosure.

## Claims

1. An anti-CD300c antibody or an antigen-binding fragment thereof that specifically binds to the extracellular domain of CD300c and inhibits the binding of translationally controlled tumor protein (TCTP) to CD300c.

2. The anti-CD300c antibody or an antigen-binding fragment thereof of claim 1, wherein
(a) the anti-CD300c antibody or an antigen-binding fragment thereof comprises:
a light chain variable region comprising a light chain CDR1 set forth in SEQ ID NO: 9 or SEQ ID NO: 34,
a light chain CDR2 set forth in SEQ ID NO: 10 or SEQ ID NO: 35, and
a light chain CDR3 set forth in SEQ ID NO: 11 or SEQ ID NO: 36; and
a heavy chain variable region comprising a heavy chain CDR1 set forth in SEQ ID NO: 12 or SEQ ID NO: 27,
a heavy chain CDR2 set forth in SEQ ID NO: 13 or SEQ ID NO: 28, and
a heavy chain CDR3 set forth in SEQ ID NO: 14, SEQ ID NO: 22, SEQ ID NO: 44, or SEQ ID NO: 51; or
(b) the anti-CD300c antibody or an antigen-binding fragment thereof comprises at least one amino acid substitution in the sequences of (a), wherein:
in the heavy chain CDR1 set forth in SEQ ID NO: 27, F at position 2 is substituted with Y, N at position 3 is substituted with T, R at position 5 is substituted with G, T, or S, N at position 6 is substituted with G or S, or A at position 8 is substituted with G;
in the heavy chain CDR2 set forth in SEQ ID NO: 28, N at position 2 is substituted with S, G at position 3 is substituted with W or V, R at position 4 is substituted with N, Y, or S, G at position 5 is substituted with N, D at position 6 is substituted with G, T at position 7 is substituted with D, N, or S, or T at position 8 is substituted with I; or
in the heavy chain CDR3 set forth in SEQ ID NO: 51, R at position 2 is substituted with S, G at position 3 is substituted with E, P at position 4 is substituted with A or D, Y at position 5 is substituted with S, Y at position 6 is substituted with SS, F at position 7 is substituted with Y, or D at position 8 is substituted with N; and
wherein the heavy chain CDR1 comprises 0 to 5 substitutions, the heavy chain CDR2 comprises 0 to 6 substitutions, and the heavy chain CDR3 comprises 0 to 4 substitutions, provided that heavy chain CDR1, heavy chain CDR2, or heavy chain CDR3 comprises at least one substitution.

3. The anti-CD300c antibody or an antigen-binding fragment thereof of claim 1, wherein the anti-CD300c antibody or an antigen-binding fragment thereof comprises:
a light chain variable region comprising a light chain CDR1 set forth in SEQ ID NO: 9 or SEQ ID NO: 34,
a light chain CDR2 set forth in SEQ ID NO: 10 or SEQ ID NO: 35, and
a light chain CDR3 set forth in SEQ ID NO: 11 or SEQ ID NO: 36; and
a heavy chain variable region comprising a heavy chain CDR1 set forth in SEQ ID NO: 12, SEQ ID NO: 20, SEQ ID NO: 27, SEQ ID NO: 37 or SEQ ID NO: 49,
a heavy chain CDR2 set forth in SEQ ID NO: 13, SEQ ID NO: 21, SEQ ID NO: 28, SEQ ID NO: 38 or SEQ ID NO: 50, and
a heavy chain CDR3 set forth in SEQ ID NO: 14, SEQ ID NO: 22, SEQ ID NO: 29, SEQ ID NO: 39, SEQ ID NO: 44 or SEQ ID NO: 51.

4. The anti-CD300c antibody or an antigen-binding fragment thereof of claim 1, wherein the anti-CD300c antibody or an antigen-binding fragment thereof comprises:
a light chain variable region set forth in SEQ ID NO: 7, SEQ ID NO: 18, SEQ ID NO: 25, SEQ ID NO: 32, SEQ ID NO: 42, SEQ ID NO: 47 or SEQ ID NO: 54; and
a heavy chain variable region set forth in SEQ ID NO: 8, SEQ ID NO: 19, SEQ ID NO: 26, SEQ ID NO: 33, SEQ ID NO: 43, SEQ ID NO: 48 or SEQ ID NO: 55.

5. The anti-CD300c antibody or an antigen-binding fragment thereof of claim 4, wherein the anti-CD300c antibody or an antigen-binding fragment thereof further comprises:
a linker set forth in SEQ ID NO: 15.

6. The anti-CD300c antibody or an antigen-binding fragment thereof of claim 1, wherein the anti-CD300c antibody or an antigen-binding fragment thereof consists of the amino acid sequence set forth in SEQ ID NO: 6, SEQ ID NO: 17, SEQ ID NO: 24, SEQ ID NO: 31, SEQ ID NO: 41, SEQ ID NO: 46, or SEQ ID NO: 53.

7. The anti-CD300c antibody or an antigen-binding fragment thereof of claim 1, wherein the anti-CD300c antibody or an antigen-binding fragment thereof comprises:
a light chain variable region comprising a light chain CDR1 set forth in SEQ ID NO: 61,
a light chain CDR2 set forth in SEQ ID NO: 62, and
a light chain CDR3 set forth in SEQ ID NO: 63; and
a heavy chain variable region comprising a heavy chain CDR1 set forth in SEQ ID NO: 64,
a heavy chain CDR2 set forth in SEQ ID NO: 65, and
a heavy chain CDR3 set forth in SEQ ID NO: 66.

8. An anti-CD300c antibody or an antigen-binding fragment thereof comprising:
a light chain variable region set forth in SEQ ID NO: 59; and
a heavy chain variable region set forth in SEQ ID NO: 60.

9. The anti-CD300c antibody or an antigen-binding fragment thereof of claim 7, wherein the anti-CD300c antibody or an antigen-binding fragment thereof consists of the amino acid sequence set forth in SEQ ID NO: 58.

10. The anti-CD300c antibody or an antigen-binding fragment thereof of claim 7, wherein the anti-CD300c antibody or an antigen-binding fragment thereof further comprises:
a linker set forth in SEQ ID NO: 15.

11. A nucleic acid molecule encoding the antibody or an antigen-binding fragment thereof of any one of claims 1 to 10.

12. An expression vector comprising the nucleic acid molecule of claim 11.

13. A host cell into which the expression vector of claim 12 is introduced.

14. A pharmaceutical composition for the prevention or treatment of allergic diseases, inflammatory diseases, or malaria, comprising a CD300c inhibitor as an active ingredient.

15. The pharmaceutical composition of claim 14, wherein the CD300c inhibitor is the antibody or an antigen-binding fragment thereof of any one of claims 1 to 10.

16. The pharmaceutical composition of claim 15, wherein the allergic disease is selected from the group consisting of asthma, bronchitis, chronic obstructive pulmonary disease, bronchiectasis, rhinitis, atopic dermatitis, urticaria, hay fever, conjunctivitis, and anaphylaxis.

17. The pharmaceutical composition of claim 15, wherein the inflammatory disease is selected from the group consisting of Rheumatoid arthritis, bronchitis, pneumonia, arthritis, nephritis, psoriasis, dermatitis, Crohn's disease, enteritis, gingivitis, arteriosclerosis, coronary arteritis, hepatitis, Behcet's disease, bladder cancer, prostatitis, pyelonephritis, glomerulonephritis, osteomyelitis, thyroiditis, uveitis, peritonitis, meningitis, and pulmonary fibrosis.

18. A drug screening method for the prevention or treatment of allergic diseases, inflammatory diseases, or malaria, comprising:
(a) treating isolated cells expressing CD300c with dimeric translationally controlled tumor protein (dTCTP) and a candidate substance;
(b) measuring the level of NF-κB signaling pathway activity or IL-8 secretion in the isolated cells treated with the candidate substance; and
(c) screening the candidate substance that reduces the level of NF-κB signaling pathway activity or IL-8 secretion by comparing the level of NF-κB signaling pathway activity or IL-8 secretion measured in step (b) above with the level in isolated cells not treated with the candidate substance.

19. A method for providing information for the diagnosis of allergic diseases, inflammatory diseases, or malaria, comprising:
(a) measuring the expression or secretion level of CD300c from an isolated biological sample; and
(b) comparing the measured expression or secretion level with the CD300c expression or secretion level of a normal control sample.

20. A composition for the diagnosis of allergic diseases, inflammatory diseases, or malaria, comprising an agent for measuring the expression or secretion level of CD300c.
